(19) Europäisches Patentamt
European Patent Office
Office européen des brevets

(11) **EP 3 193 726 B1**

(12) **EUROPEAN PATENT SPECIFICATION**

(45) Date of publication and mention
of the grant of the patent:
**08.09.2021 Bulletin 2021/36**

(51) Int Cl.:
*A61B 8/00* *(2006.01)*  *A61B 8/08* *(2006.01)*
*A61B 8/12* *(2006.01)*  *A61B 5/00* *(2006.01)*

(21) Application number: **15842691.6**

(22) Date of filing: **16.09.2015**

(86) International application number:
**PCT/US2015/050404**

(87) International publication number:
**WO 2016/044411 (24.03.2016 Gazette 2016/12)**

(54) **IDENTIFYING ANATOMICAL STRUCTURES**

IDENTIFIZIERUNG VON ANATOMISCHEN STRUKTUREN

IDENTIFICATION DE STRUCTURES ANATOMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.09.2014 US 201462051670 P**

(43) Date of publication of application:
**26.07.2017 Bulletin 2017/30**

(73) Proprietor: **Avaz Surgical, LLC
Chicago, Illinois 60611 (US)**

(72) Inventors:
• **SINGH, Kern
Chicago, Illinois 60611 (US)**
• **GUPTA, Sachin
Hinsdale, Illinois 60521 (US)**

(74) Representative: **V.O.
P.O. Box 87930
Carnegieplein 5
2508 DH Den Haag (NL)**

(56) References cited:
**US-A1- 2003 125 629    US-A1- 2005 245 822
US-A1- 2008 183 076    US-A1- 2011 319 765
US-A1- 2012 197 124    US-A1- 2012 310 064
US-A1- 2012 310 064    US-A1- 2013 085 394
US-A1- 2013 172 742    US-A1- 2013 172 742
US-A1- 2014 018 668    US-A1- 2014 018 668
US-B1- 6 248 072       US-B1- 6 741 895
US-B2- 6 530 888       US-B2- 8 663 116**

**Description**

BACKGROUND

Surgical techniques utilizing minimally invasive surgery ("MIS") are being rapidly adapted to replace current traditional "open" surgical procedures. "Open" procedures typically require larger skin incisions that may cause significant collateral damage to uninvolved anatomic structures. For example, intervening soft tissue (e.g., tendons, ligaments, facet capsules, muscles, and so on) may be cut and even potentially excised to allow for direct surgical visualization of the operated-upon area or anatomical structure.

In contrast, minimally invasive techniques, which may also be referred to as "percutaneous" techniques, involve significantly smaller incisions and are less traumatic to the patient's anatomy. Soft tissues may be preserved with minimal collateral damage to the uninvolved anatomy. Typical benefits of MIS may include decreased blood loss, decreased postoperative pain, smaller scar formation, decreased cost, and a faster rehabilitation for the patient than in to "open" or conventional surgical techniques.

Minimally invasive surgery techniques are currently being adapted to a variety of surgical procedures. For example, minimally invasive techniques in the form of laparoscopic procedures, such as a laparoscopic colectomy for carcinoma of the colon, have been developed. More recently, surgeons have utilized MIS in spinal surgery applications.

It is noted that US 2012 / 197124 A1 discloses an apparatus according to the preamble portion of the appended independent claim 1.

BRIEF SUMMARY

Present MIS techniques are unable to accurately and consistently detect and avoid key anatomical features, such as neural elements, potentially resulting in profound neurological sequelae and deleterious impacts to other systems. For example, even a minimally invasive surgical instrument, if impacting or contacting with nervous system elements (e.g., nerves, spinal cord) may result in loss of sensation, sensory overload, pain, or other unwanted or harmful effects. Detection and identification of anatomical features may assist in combating these problems and other problems that may become apparent upon reading of this disclosure.

Accordingly, the invention provides an apparatus according to the appended independent claim 1, a method according to the appended independent claim 11 and a non-transitory computer readable medium according to the appended independent claim 17. Preferable embodiments of the invention are provided by the appended dependent claims 2-10, 12-16 and 18-20.

Accordingly, in one aspect of the present disclosure, a device may be provided for minimally invasive surgery and may include a body comprising a proximal portion, a distal portion, and a main portion formed between the proximal portion and distal portion. At least one ultrasound transducer may be arranged at the distal portion of the body and may be configured to scan a region extending away from the main portion of the body. The device may include a signal processing unit having at least one processor and memory storing instructions that cause the at least one processor to receive a signal from the at least one ultrasound transducer and identify an anatomical structure based on the signal.

In another aspect, a device may be provided for minimally invasive surgery. The device may include a proximal portion, a distal portion, a main body formed between the proximal and distal portions of the device having a longitudinal axis and at least one ultrasound transducer disposed within the distal portion of the device and configured to scan a region adjacent to a distal end of the distal portion of the device.

In another aspect, a method may be provided and may include receiving data from at least one ultrasound transducer arranged at a distal portion of a body and configured to scan a region extending away from a main portion of the body. The method may also include processing the data to identify an anatomical structure located within the region, and outputting an indication associated with the anatomical structure.

In another aspect, a method for identifying a target anatomy may be provided with a device having a distal portion and at least one ultrasound transducer at least partially disposed within a main body of the device. The method may include scanning an anatomy of a patient anatomy for the target anatomy, determining a voltage trace of the patient's anatomy, comparing the voltage trace of the patient's anatomy to a predetermined voltage trace of the target anatomy, and sending a notification if the voltage trace of the patient's anatomy matches the predetermined voltage trace of the target anatomy.

BRIEF DESCRIPTION OF THE DRAWINGS

Figure 1 is a side view of one embodiment of a device.
Figure 2 is a side view of another embodiment of the device of Figure 1.
Figure 3 is a functional diagram of the ultrasound imaging system that may be used in one embodiment of the present disclosure.
Figure 4 is a diagram of an ultrasound transducer that may be used in one embodiment of the present disclosure.
Figure 5 is another functional diagram of one embodiment of the ultrasound imaging system that may be used in an embodiment of the present disclosure.
Figure 6 is one embodiment of the device having more than one ultrasound transducer disposed therein.

Figure 7 is another embodiment of the device having one ultrasound transducer disposed therein.

Figure 8 depicts the scanning width of a transducer.

Figure 9 depicts the scanning width of a transducer in one configuration.

Figure 10 is another embodiment of the present disclosure where one of the transducers is positioned at an angle with respect to the other transducer.

Figure 11 depicts the scanning width of the embodiment of Figure 10.

Figure 12 is yet another embodiment of the present disclosure where two transducers are angled towards the longitudinal axis of the device.

Figure 13 depicts scan images of a target anatomy taken by one embodiment of the present disclosure.

Figure 14 is a scan and A-line image scan of the target anatomy captured by one embodiment of the present disclosure.

Figure 15 depicts the configuration of one embodiment of the present disclosure.

Figure 16 depicts images of the target anatomy captured by one embodiment of the present disclosure.

Figure 17 depicts additional images of the target anatomy captured by one embodiment of the present disclosure.

Figure 18 is a scan of the target anatomy captured by one embodiment of the present disclosure.

Figure 19 depicts one embodiment of a retractor system that can be used with embodiments of the present disclosure.

Figure 20 depicts one embodiment of the dilator system that can be used with embodiments of the present disclosure.

Figure 21 is one embodiment of the present disclosure that is incorporated into a glove.

Figure 22 is a partial side view of the embodiment disclosed in Figure 21.

Figure 23 is a front cross-sectional view of one embodiment of the glove embodiment disclosed in Figure 21.

Figure 24 is a front cross-sectional view of another embodiment of the glove embodiment disclosed in Figure 21.

Figure 25 is a front cross-sectional view of yet another embodiment of the glove embodiment disclosed in Figure 21.

Figure 26 is diagram of another embodiment of the present disclosure that utilizes Optical Coherence Tomography.

Figure 27 is one embodiment the probe used with the embodiment disclosed in Figure 26.

Figure 28 depicts two embodiments of the probe used with the embodiment disclosed in Figure 26.

Figure 29 is a side view of one embodiment of the present disclosure having direct visualization capability.

Figure 30 is a cross-sectional view of the embodiment disclosed in Figure 29.

Figure 31 is another embodiment of the present disclosure having direct visualization capability.

Figure 32 is a partial cross-sectional view of the embodiment disclosed in Figure 31.

Figure 33 is a partial front cross-sectional view of the embodiment disclosed in 29.

Figure 34 is another embodiment of the present disclosure disclosed in Figure 29.

Figure 35 is a side cross sectional view of another embodiment of the present disclosure disclosed in Figure 31.

Figure 36 is yet another embodiment of the channel disclosed in Figure 29.

Figure 37 is yet another embodiment of the channel disclosed in Figure 29.

Figure 38 depicts one embodiment of the present disclosure in use with a retractor system.

Figure 39 depicts one embodiment of the retractor system of the preset invention.

Figure 40 depicts another embodiment of the retractor system of the preset invention.

Figure 41 depicts an illustrative graph of Scan line energy as a nerve discriminant.

Figure 42 depicts an illustrative user interface design window containing a designed bandpass filter and specifications.

Figure 43 depicts an illustrative application of a bandpass filter on the received RF signals, thereby suppressing low frequency noise near the transducer surface.

Figure 44 depicts examples of SVM classification schemes for separable and non-separable data.

Figure 45 depicts a plot matrix showing two-dimensional (2D) relationships between the feature variables.

Figure 46 depicts an illustrative receiver operating characteristic (ROC) curve defining performance of the trained SVM.

Figure 47 illustrates a computing device in accordance with one or more aspects described herein.

DETAILED DESCRIPTION

To help understand the present disclosure, the following definitions are provided with reference to terms used in this application.

Throughout this specification and in the appended claims, when discussing the application of aspects of the present disclosure with respect to the body's tissue, spine or other neural elements, the term "proximal" with respect to such a device is intended to refer to a location that is, or a portion of the device that is, closer to the operator. The term "distal" is intended to refer to a location that is, or a portion of the device, further away from the operator.

The embodiments below are described with reference to the drawings in which like elements are referred to by like numerals. The relationship and functioning of the various elements are better understood by the following detailed description. The embodiments as described below are by way of example only and the present disclosure is not limited to the embodiments illustrated in the drawings.

According to one or more aspects of the present disclosure, a device capable of detecting target anatomical structures may be provided. The device may utilize ultrasound and/or Optical Coherence Tomography (OCT) technology as the device is being advanced through a patient's anatomy. The device may have a distal portion having a tip, where the tip can be used to dissect a patient's anatomy without puncturing or tearing the patient's anatomy and while simultaneously allowing the device to inspect the anatomy as it is being dissected by the tip. While the device discussed herein is discussed in the context of a device that can be held by an operator, it is contemplated that the device and/or parts of the device may be used during automated procedures such as those being performed by robotic and other similar systems.

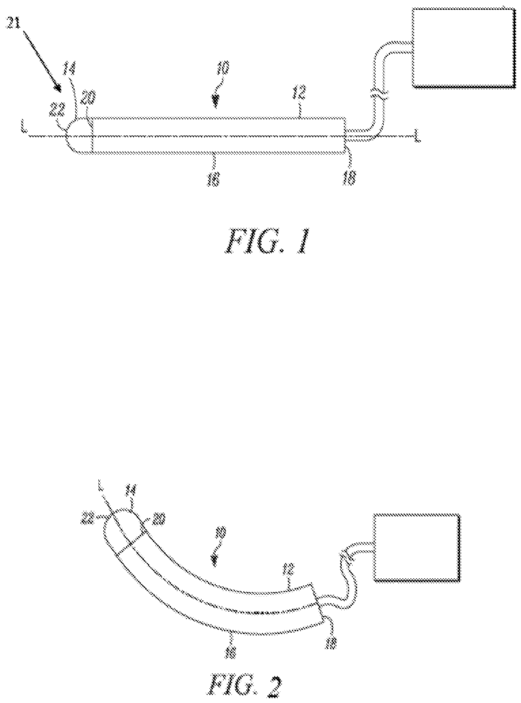

In one embodiment, shown in Figure 1, the device 10 has a proximal portion 12 and a distal portion 14 with a main body 16 disposed between the proximal and distal portions 12, 14. The main body 16 has a proximal end 18 and a distal end 20 and is defined by a longitudinal axis L. The proximal end 18 may have a handle (not shown) or gripping portion (not shown) attached thereto. The length of the main body 16 may vary, but can include a length of 50 to 300 mm; however, in some embodiments the length may fall outside of this range. Similarly, the outer diameter of the main body 16 may vary and can include an outer diameter of between 3 mm and 20 mm. The main body 16 can be made out of any preferable surgical grade material, including but not limited to, a medical grade polymer including PEEK (polyether ether ketone), stainless steel, carbon fiber, and titanium. The main body 16, and one or more of the components of the device 10 generally, may contain radio-opaque markers to allow an operator to detect the location of the device 10 with respect to the anatomy of a patient via radiographic imaging.

As shown in Figure 1, the distal portion 14 of the device 10 includes a tip 22. The tip 22 may be hemispherical in shape, as illustrated in Figure 1, but it is contemplated that the tip 22 may also be of a different shape. For example, and without limitation, the tip 22 may have a semi-spherical, conical, pyramidal, spear or aspherical shape. The tip 22 may be configured to dissect a patient's anatomy, such as a muscle, without tearing or disrupting the patient's anatomy as it passes through the tissue. As a result, the outer diameter of the tip 22 may have a diameter ranging anywhere between 1 mm and 50 mm and preferably between 2 mm and 9 mm. It is appreciated that the outer diameter of the tip 22 may fall outside of this range as well. It is further appreciated that the tip 22 is optional such that particular embodiments of the device 10 may not include the tip 22.

As illustrated in the embodiment shown in Figure 1, the main body 16 of the device 10 may be substantially straight. However, it is contemplated that the main body 16 may have different shapes, including having a curved shape with a non-zero radius of curvature. An example of such an embodiment is illustrated in Figure 2, which may be used for MIS requiring access through the presacral space of a patient. The main body 16 may also take on an "L", "C", "U" shape or a shape therebetween.

The device 10 may include ultrasonic capability. A purpose of this device may be to serve as an instrument that features a specifically patterned array of high frequency ultrasound transducers and a monitoring system that collects spectral properties of specific tissue in the body. For example, the system may be able to detect the spectral properties of muscle, fat, nerve and bone. As the anatomy is stimulated by the ultrasound transducer(s), it will emit a specific spectral property that can be detected by the monitoring system. The system may examine scan line images and seek specific parameters of amplitude, shape, and other spectral content in order to differentiate the signals coming from the nerve and signals coming from surrounding tissues. For example, nerve tissue may be hypoechoic as compared with the surrounding tissue. However, there are internal structures that provide features in the signal that identify the nerve from single scan lines or RF properties. The system will inform the operator that the device is adjacent to or proximate to the specific type of anatomy that is detected by the system. The device can allow a surgeon to identify and avoid certain portions of a patient's anatomy (e.g. nerve) when performing a minimally invasive procedure.

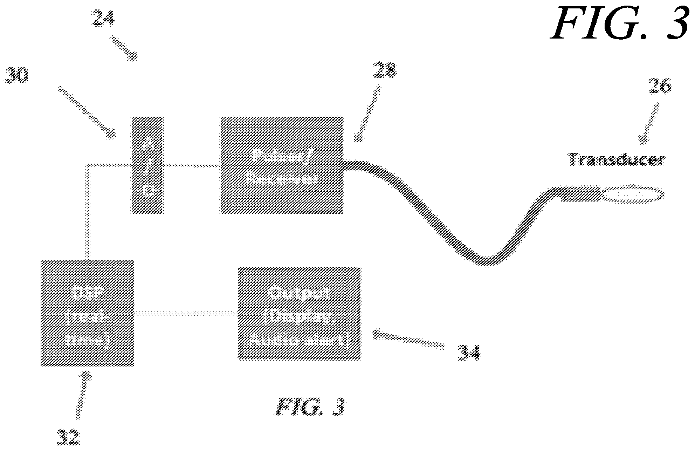

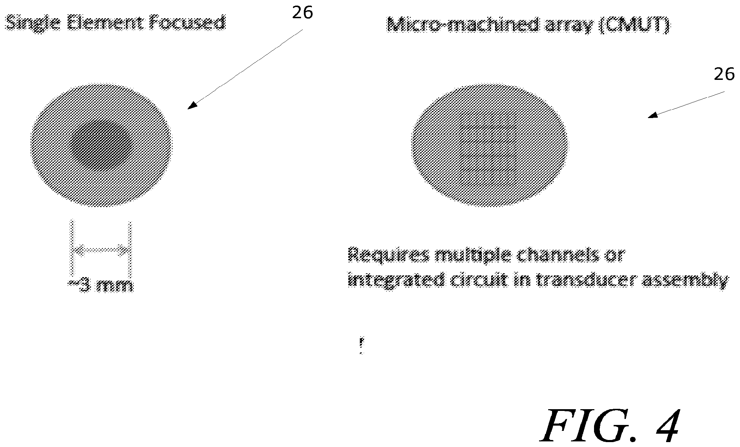

The device 10 may be equipped with ultrasound imager 24 to detect a patient's anatomy as shown in Figures 3 - 5. The ultrasound imager 24 may include a transducer 26 that is configured to emit sound waves may be disposed at the distal end 20 of the device 10. As shown in Figure 4, the transducer 26 may include a single element focused transducer, and may have a frequency operation range that includes an operating range of approximately 10-40 MHz. In some aspects, the operating range may be higher or lower than this range of frequencies. Additionally or alternatively, transducer 26 may include a micro machined array (CMUT) having multiple channels. The desirable frequency may vary depending on the application and target anatomy. For example, in one embodiment a frequency or range of frequencies may be selected for detecting nerve from surrounding tissues and adjacent anatomy on b-mode images based on image texture and echogenicity. Reliably distinguishing between nerve and muscle tissue in real time may require quantitative approaches, and may require automated or manual calibration of the system to estimate tissue-specific properties. In some aspects, a meta-analysis comparing ultrasound to nerve-stimulation may result in superior outcomes for ultrasound guidance.

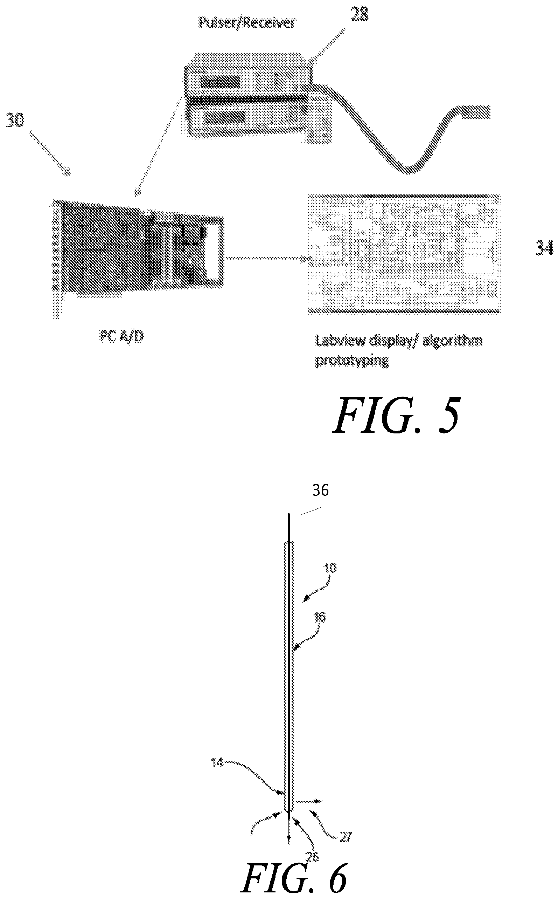

As shown in Figures 3 and 5, the transducer 26 may be in communication with a RF-pulser/receiver 28, which may be in communication with an analog to digital converter 30, which may be in communication with a digital signal processor 32 and an output 34 such as a monitor.

In one embodiment, the transducer 26 converts an electric signal or pulse generated from the RF-pulser/receiver 28 into a sound wave and then converts a reflected sound wave back into an electrical signal. The ultrasound transducer 26 launches sound pulses, which may be short, high-frequency non-damaging sound pulses, into the tissue, and then may wait to hear the reflection from the tissue. Since the speed of sound in tissues is high (~1500 m/s), this process may take a few milliseconds to image a few millimeters of tissue. As referenced above, the RF-pulser/receiver 28 may generate an electrical impulse that may be sent (e.g., via a cable) to the transducer 26 to generate a sound wave, and may also receive a signal from the transducer 26 generated by the reflected sound waves that the transducer receives 26. The analog to digital converter 30 converts the analog radiofrequency signal received from the transducer 26 into a digital form that a computer may analyze. The digital signal processor 32 processes the digitized signal received from the digital converter 30. Signal filtering and processing operations may programmed into the hardware, firmware, and/or software of various components of the system (e.g., the digital signal processor 32) to detect the reflected signal properties of the tissue and distinguish between nerve and muscle tissues in real-time. Once a nerve tissue signature is detected, a hardware, firmware, and/or software system may communicate with the output display 34. The output 34 may include a visual monitor that may be programmed to display the anatomy or signals indicative of the anatomy (e.g., via actual images or programmable color configurations (red/yellow/green)) and/or a sound-generating device which may be controlled to emit an audible indicator (e.g. alarm or a "beep"). For example, the sound-generating device, such as a speaker, may emit a "beep" when the device 10 encounters/detects the presence of target anatomy (e.g. nerve) within a predetermined range (e.g. 1 mm to 10 cm).

It is appreciated that one or more of these components may be in wireless communication with one another, may be combined into one or components, and other additional components may be in communication between each of these components or one or more identified components and may not be included in a particular embodiment.

In one embodiment, the outer diameter of the transducer 26 may be approximately 3 mm, but may range anywhere between approximately 1 mm and 10 mm. Further, the transducer 26 is configured to be disposed in a variety of locations with respect to the device 10. For example, as shown in Figure 6, the transducer 26 may be disposed at the distal end 20 of the main body 16 or at the tip 22 portion of the distal end 20. The transducer 26 may also be removable such that it can be removably disposed within a conduit 36 formed within the device 10 and removed once a working space is identified and accessible. In some aspects, the working space may be the space created by insertion and/or manipulation of the device 10 within the patient's anatomy.

Multiple transducers 26 may be provided as part of the device 10. In some aspects, two or more transducers 26 may be side positioned (e.g. on either side of the main body 16) so as to provide for multi-directional scanning of the patient's anatomy to detect the nerve. The side positioned transducers may be configured to scan the anatomy around in a circumferential direction around the main body 16, and may detect the nerve (or other target anatomy) that was not detected by a transducer positioned at the distal end 20 of the main body 16. The multi-directional scanning enables the system to generate a scan image of the patient's anatomy in multiple directions as the device 10 is advanced through the patient's anatomy.

Returning to Figure 6, the device 10 may include at least one ultrasound transducer 26 (such as a high frequency ultrasound transducer) that is used to stimulate a patient's anatomy, such as muscle, fat, nerve, and bone, and so on. A series of transducers 26 (e.g., a transducer, two or more transducers) may be disposed along the length of the device 10 to allow for a wider pattern of ultrasonic stimulation of the surrounding anatomy. In this embodiment, there may one transducer 26 on the distal end of the device 10 that emits an ultrasonic frequency in a direction that is substantially parallel to the longitudinal axis of the device 10. There may be another transducer 26 adjacent to the first transducer 27 that emits ultrasonic frequency along a path that is substantially perpendicular to the longitudinal axis of the device 10. It can be appreciated that the transducers 26 can be orientated in any direction that is required for the particular application.

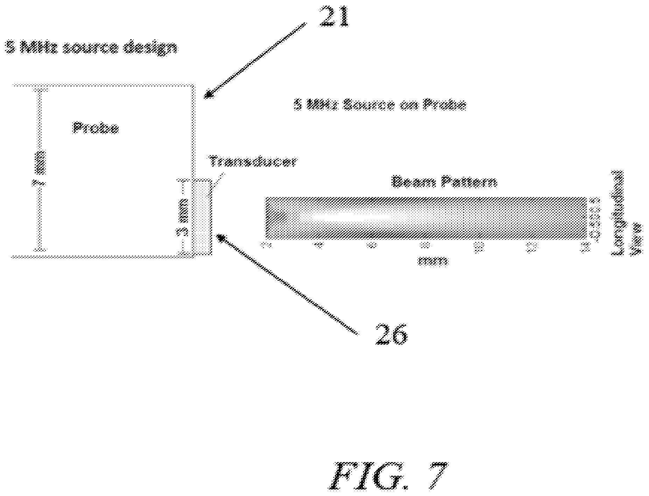

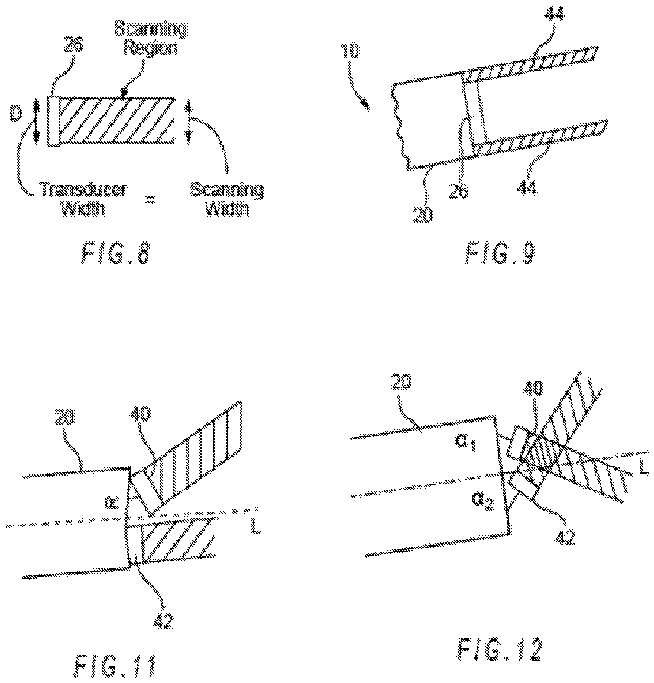

Figure 7 depicts one embodiment where a 5 MHz transducer 26 is located on the distal end 21 of the device. In such an embodiment, the diameter of the transducer 26 may be 3 mm and the transducer may be forward facing. In such and embodiment, the scanning range is approximately 14 mm. In some aspects, an area of the scanning region (hatched section in Figure 8) by a transducer 26 may not exceed the outer diameter of the transducer. This may be because the scanning width is circumscribed by the outer diameter of the transducer 26 and the peripheral limitations of the transducer 26 such that the transducer 26 cannot identify or scan a region that lies beyond of the diameter of transducer 26.

Accordingly, in some aspects such as those disclosed herein where the transducer 26 is housed within a device 10 the transducer 26 may be unable to scan the region that is directly front of (distal to) the outer portions of the device that house the transducer 26. This region 44 is depicted in Figure 9. One result of this is that the target anatomy may go undetected if it is positioned beyond the scanning region of the transducer 26.

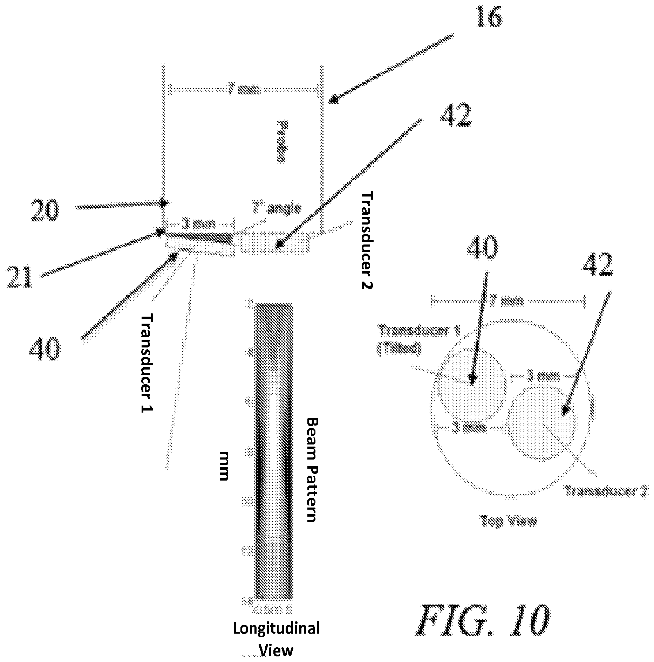

In some aspects, for example to detect target anatomy that lies just beyond the scanning region of the transducer 26 (i.e. outside of the scanning diameter), the device 10 may include two or more transducers positioned at an angle relative to one another. For example, as shown in Figures 10 and 11, the first transducer 40 may be positioned at an angle with respect to the outer edge of the main body 16. The angle may be measured from the face of distal end 21 of the device 10, or may be measured from the horizontal axis that intersects the longitudinal axis of the device 10. The angle $\alpha$ in this embodiment is 7° but it is appreciated that it can vary from 0° to 180° depending on the particular embodiment. Further, in this embodiment, the angle may be formed between the edge of the transducer 26 and the adjacent outer edge of the main body 16.

Transducer 40 may be angled with respect to any portion of the main body 16. For example, the first transducer 40 may be angled by pivoting the first transducer 40 about the portion positioned along, or closest to, the longitudinal axis of the main body 16 as shown in Figure 12. Specifically, as shown in Figure 12, the transducers 40, 42 may be angled towards the longitudinal axis of the main body 16 such that the angle of tilt, $\alpha_1$ $\alpha_2$, are measured as the angle of the transducer from the longitudinal axis of the main body 20.

In the embodiment shown in Figure 10, the first transducer 40 may be positioned at an angle (e.g., 7° from the edge of the main body 16) allowing the first transducer 40 to scan a region extending beyond the outer edge of the main body 16. Angling the first transducer 40 may allow the device 10 to scan and detect any target anatomy residing outside of the scanning region of a transducer that is not angled with respect to the main body 16 of the device. A region that may be scanned by the first angled transducer 40 in this particular embodiment is shown in Figure 10.

The two transducer elements may fit on the tip of a device 10. This may allow, for example, the device 10 to detect backscattered signals. In some aspects, the device 10, and/or the two transducer elements may be configured to detect the presence of nerves or other neural elements existing distally up to 1 cm from the probe tip 22 (e.g., the nerves or neural elements may be in the pathway of the device). In some aspects, a device may be provided with transducers having different stimulating frequencies (e.g., a first transducer 40 may stimulate with a frequency of 5MHz, and a second transducer may stimulate with a frequency of 10 MHz). Beam patterns or fields emitting from the sources may be modeled using field II assuming an element diameter of 3 mm and a focal number of 3 (f/3).

In some aspects, two transducer elements 40, 42 may be positioned at the tip of the probe. The tiled element 40 faces outward with an angle of tilt, such as 7°. The tiled element may have one end at the edge of the probe. The un-tilted element 42 may be centered from the edge of the distal portion of the device 10. This configuration may allow the device 10 to be rotated as it is snaked through the tissue so that the cross sectional surface area of the probe may be at least 1 cm above the probe surface.

The diameter of the transducers 40, 42 may vary and can range from 1 mm to 20 mm. For example, in the embodiment shown in Figure 10, the first transducer and second transducers 40, 42 each have a diameter of approximately 3 mm. It is not necessary for the transducers to have the same diameter of one another and can be staggered as shown in the top view of Figure 10.

The main body 16 can be rotated so as to allow the first transducer 40 to scan the entire outer region to detect whether any target anatomy is present that is just beyond the scanning area of a forward facing transducer. By rotating the main body 16 about its longitudinal axis, the first transducer 40 can scan the outer region that does not fall within the scanning region of a transducer that is not angled with respect to the main body 16 of the device.

The number of angled transducers may vary and can be positioned at various angles with respect to the distal end of the main body 16. For example, as shown in Figure 12, the first and second transducers 40, 42 are positioned toward one another so that their scanning areas cross to provide a scan of the area distal to the distal portion 20 of the device 10 and a region beyond the area directly in front of to the outer diameter of the transducers 40, 42 as shown in Figure 9. Also, the transducers 40, 42 may be positioned at an angle on more than one axis with respect to the distal end of the main body 16.

The device 10 can be configured to determine the b-mode scans of the patient's anatomy and associated data, including, for example, the voltage trace from a scan line in the b-mode image. The voltage trace for certain anatomical parts (e.g., a nerve) may have a unique voltage trace that may be used to detect like anatomical parts within the patient's anatomy. One way to detect like anatomical parts may be by comparing the voltage trace from a scan line of a b-mode image to the known voltage trace from the scan line of the target anatomy. Specifically, the b-mode scans (and associated data, such as a-scan lines, voltage traces, and the like) may be captured by the device 10. The scans and/or data may be compared to the pre-determined b-mode scans (and associated data) of known anatomical features (e.g., nerve) to determine whether the region captured by the b-mode scan from the device 10 contain the target anatomy.

The device 10 may be used in conjunction with a neuromonitoring system capable of detecting certain portions of a patient's anatomy, including neural elements that include a nerve, nerve bundle, or nerve root. For the purposes of this discussion, the device 10 and neuromonitoring system will be discussed with respect to detecting a patient's spinal nerve but it is contemplated that the device 10 and neuromonitoring system can be used to detect other nerves (peripheral and central) as well as the spinal cord. One type of neuromonitoring system that can be used in conjunction with the device 10 is disclosed in U.S. Patent No. 7,920,922.

Experimentation

The discussion below is directed to the experiment used to determine the target ultrasonic frequency that can be used to detect nerve using the device 10 and whether the b-mode scan images captured by the device 10, which is inserted into the patient's anatomy, is comparable to results captured by traditional non-invasive ultrasound devices. Both target objectives were accomplished using the following process.

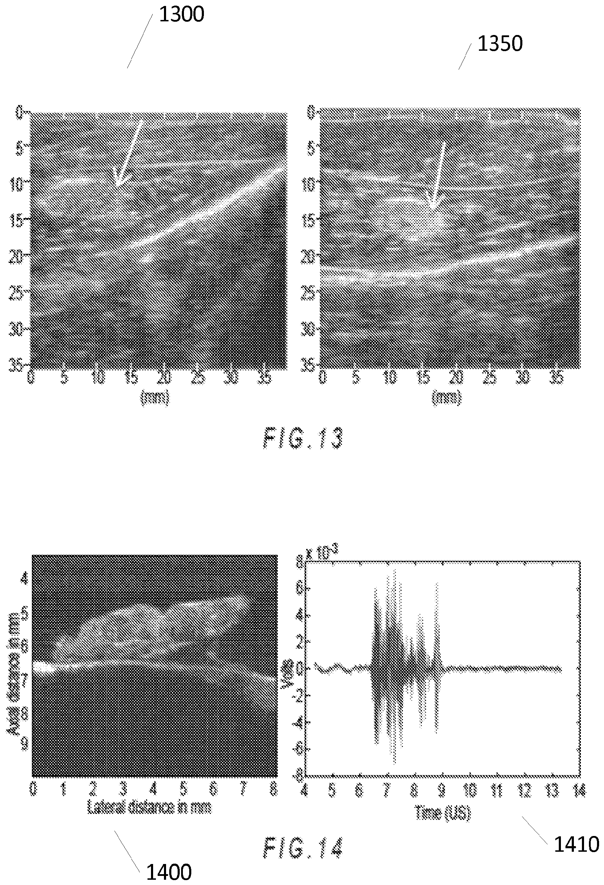

Figure 13 illustrates exemplary data indicative of a scan of a sciatic nerve of a rabbit with a clinical ultrasound array system. The scan was performed before and after euthanizing the rabbit to be sure that the nerve could be seen in both cases. Figure 13 depicts b-mode images (nerve cross-sectional view) for alive (left, image 1300) and dead (right, image 1350).The nerve may be seen in each case (arrow 1305, arrow 1355).

The nerve was scanned using a high frequency (40 MHz) probe with the bottom of the nerve still attached to the muscle and the nerve centered in the probe's depth of field. Figure 14 illustrates a b-mode image of this scan. The image shows (from top to bottom): water (1400), nerve (1410), and muscle (1420). The nerve separates from the muscle towards the right side of the image, and you can see a gap between the nerve and the muscle. Figure 14 also illustrates a plot 1450 depicting the voltage trace from a scan line in the center of the b-mode image, indicated by the vertical line.

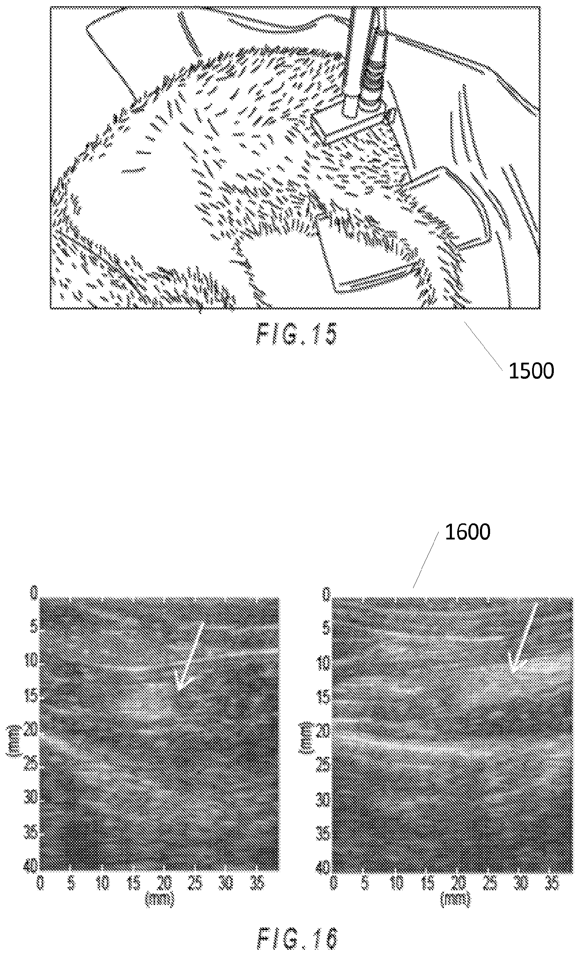

The hind limb sciatic nerve was scanned with a 20 MHz single-element probe though the leg muscle. The muscle was kept intact, and removed the skin to provide a window to see into the muscle. The image 1500 shown in Figure 15 illustrates the setup.

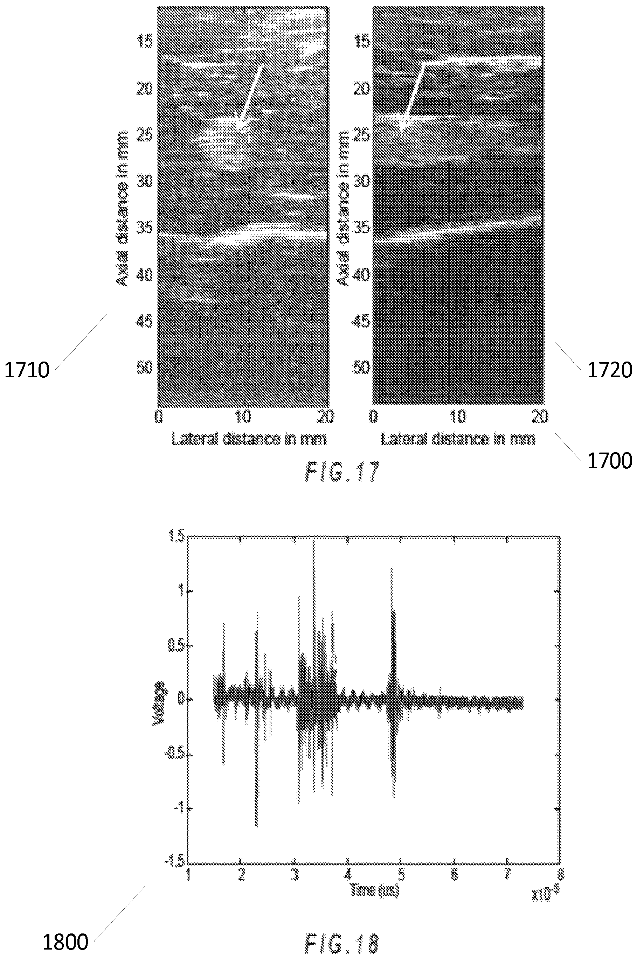

A clinical scan was performed before scanning with the 20 MHz probe. An image 1600 from the clinical scan of the nerve is shown in Figure 16, and an image 1700 from the scan performed by the 20MHz probe is shown in Figure 17. A comparison between the clinical imaging system and the 20 MHz probe system suggests that both techniques produce similar images. For example, the cross-section and length-wise (with respect to the long direction of the nerve) scan planes both show the nerve in the background muscle for both the clinical system and the 20 MHz system.

The 20 MHz results are important for at least two reasons: First, the results show that the contrast inside the muscle exists at 20 MHz as demonstrated in the left image 1710 in Figure 17. Second, the depth of penetration for the 20 MHz signal was sufficient to be seen at more than 1 cm of depth. This may be the distance away from the surgical probe required for detecting the nerve. Therefore, this suggests that if the signals can be used to detect the nerve, the signal strength and penetration should not be an issue at the chosen ultrasound frequencies.

Figure 18 depicts an image 1800 of a single scan line through the nerve. There are characteristic signatures from the nerves that can be used to detect the nerve from a single scan line.

In another experiment, a dual element transducer 40, 42, having a configuration similar to the embodiment disclosed in Figure 10 was utilized. This embodiment was used testing of sciatic nerves of 22 rabbit legs post-mortem. A total of 142 sets of radio frequency (RF) data were collected. Each slice of data was recorded over a 30 mm lateral distance capturing an axial region of approximately 5 - 20 mm from the transducer surface. Imaging performance was evaluated when conducting a sector scan of - 35° to 35° at a depth of up to 1.5 cm and a center frequency of up to 15 MHz. The ultrasonic transducer used for scanning was dual-element, 10 MHz transducer, where each element was 3 mm in diameter with an f-number of 3. Images were acquired using a Pulser/Receiver, the settings of which are shown in the table below.

Table 1: Pulser/Receiver settings

| Pulse Repetition Frequency | 200 MHz |
| --- | --- |
| Energy | 12.5 uJ |
| Damping | 25 Ohms |
| High Pass Filter | 1 MHz |
| Low Pass Filter | 20 MHz |
| Input Attenuation | 0 dB |
| Output Attenuation | 0 dB |
| Gain | 20 dB |

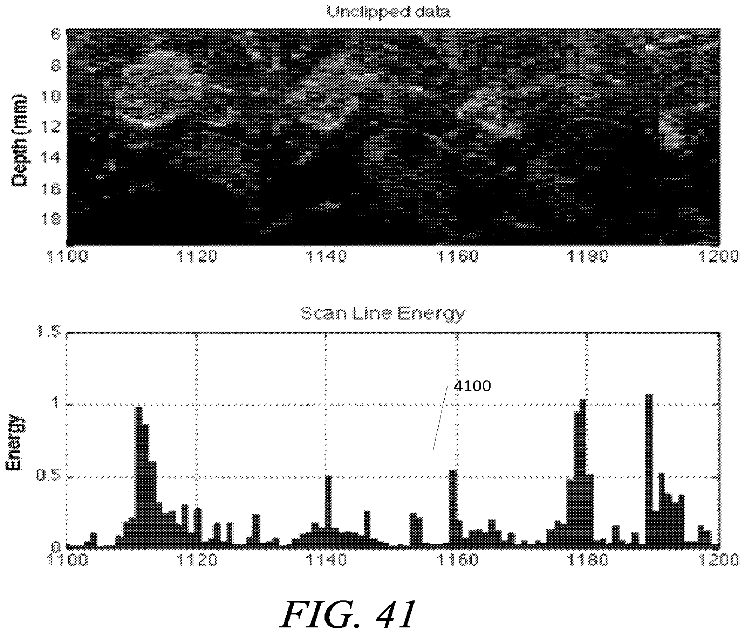

As discussed above, the device as used to capture an ultrasonic B-mode image, where the sciatic nerve may be identified as an isolated, hyperechoic region, usually elliptical or circular in nature as seen below. As such, one of the first attempts to classify the images was based on the thresholding on the energy of the received scan line. From the data displayed in Figure 41, for example, it may be seen that the scan line energy does allow for the detection of the presence of the sciatic nerve. Using energy alone, however, as a classification criterion may not be adequate to distinguish between the diffuse, weak signature of the nerve with the sharp, strong signature of an isolated strong scatterer (e.g., data point 4100 in Figure 41).

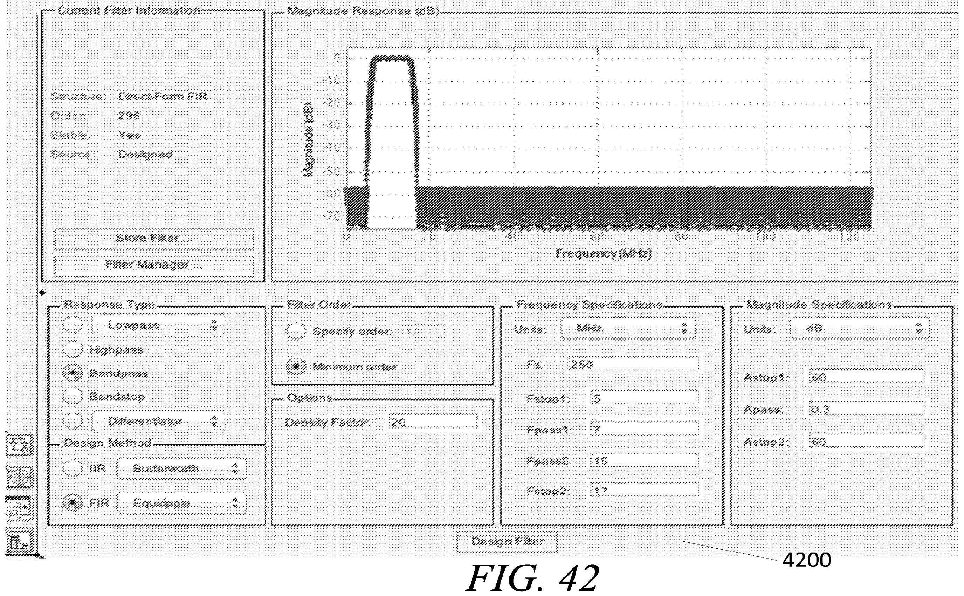

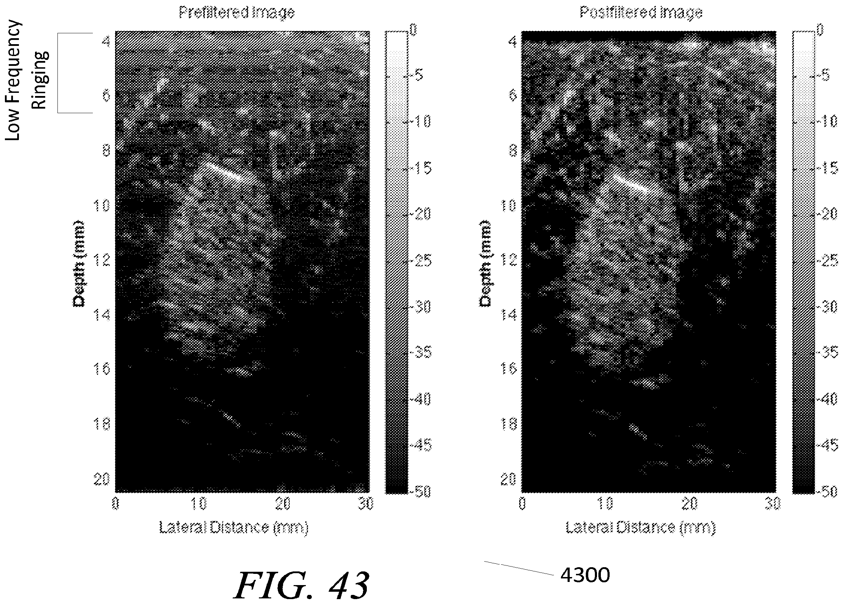

Low frequency ringing may also be present in the received RF scan lines, particularly near the surface of the transducer. Spectral analysis of the ringing may indicate a high noise component, such as a noise component between 500 kHz and 2 MHz. To combat this noise, the received RF may be passed through a finite impulse response (FIR) bandpass filter, such as software, hardware, and/or firmware configured using the design specifications described in Table 2 and illustrated in example user interface 4200 of Figure 42. Furthermore, an illustrative output 4300 of the bandpass filter is shown in Figure 43.

Table 2: Bandpass filter design specifications

| Type | Optimal Equiripple |
|---|---|
| Order | 296 (minimum order) |
| Sample Frequency | 250 MHz |
| Fstop1 | 5 MHz |
| Fpass1 | 7 MHz |
| Fpass2 | 15 MHz |
| Fstop2 | 17 MHz |
| Astop1 | 60 dB |
| Apass (ripple) | 0.3 dB |
| Astop2 | 60 dB |

Since classification of the nerve using only the scan line energy may not yield satisfactory results, it is appreciated that a multivariate approach classification approach can be explored. One commonly used multivariate classification algorithm is a support vector machine (SVM). A SVM may be a supervised learning algorithm that attempts to find an optimally separating hyperplane between two labeled sets of data. Each observation within the data sets consists of a number of features, which may be in some aspects descriptive variables that may be used to help classify the data.

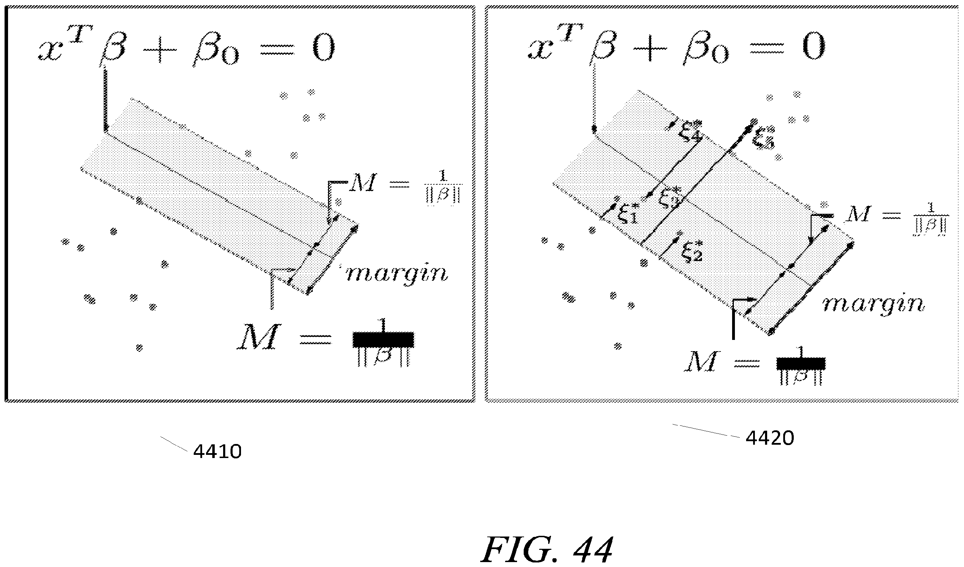

For example, with reference to SVM classification schemes 4410 and 4420 in Figure 44, consider the two-dimensional classification problem below. The $i^{th}$ observation in the training set is associated with a feature vector $x^{(i)} = (x_1^{(i)}, x_2^{(i)})$ and a data label $y^{(i)} \in \{-1, 1\}$ which describes the observation's class. Any hyperplane over the feature space may be defined as $\{ x : f(x) = \beta^T x + \beta_0 = 0 \}$, where $\beta$ is a vector. The general goal of the SVM is to solve the optimization problem

$$\min_{\beta, \beta_0} \|\beta\|$$

subject to the constraint $y^{(i)}(\beta^T x^{(i)} + \beta_0) \geq 1$

for all i in the training set. By solving this optimization problem the SVM may locate the hyperplane which maximizes the margin between separable data. Once the values for $\beta$ and $\beta_0$ are found, new events may then be classified based on which side of the hyperplane they lie, or equivalently:

$$\hat{y}(x) = \text{sign}(\beta^T x + \beta_0)$$

This simple form of SVM works for separable data, like the case in Figure 44 on the left (e.g., scheme 4410). However, in many cases the data may not be separable, such as the case on the right (e.g., scheme 4420). In this case the SVM must change the optimization problem to include *slack variables,* $\xi_i$, into the optimization problem. The new optimization problem is then given by

$$\min_{\beta, \beta_0, \xi_i} \frac{1}{2} \|\beta\|^2 + C \sum_i \xi_i$$

such that

$$y^{(i)}(\beta^T x^{(i)} + \beta_0) \geq 1 - \xi_i$$

By adding these slack variables, the constraint is now much less restrictive, since the slack variables allow for particular data points to be misclassified. The amount of misclassification may be controlled by the C reweighting factor known as the box constraint. In some aspects, the box constraint may be a parameter specified by the operator. When the box constraint is high, the optimization algorithm may force the slack variables to be small, thus resulting in a more restrictive classification algorithm.

Some classification problems do not automatically lend themselves to simple linear decision boundaries. In these cases, a feature set may be transformed into a different domain before attempting linear separation. For example, the instances of *x* may be replaced with the transformed version *h(x)*. Typically these feature transformations are specified by their kernels. The kernel of the transformation is defined as the inner product between transformed feature vectors, or symbolically,

$$K(x^{(i)}, x^{(j)}) = \langle h(x^{(i)}), h(x^{(j)}) \rangle$$

A commonly used kernel may be the radial basis function or Gaussian kernel, which may have the form

$$K(x^{(i)}, x^{(j)}) = \exp(-\frac{\|x^{(i)} - x^{(j)}\|^2}{2\sigma})$$

In practice, Gaussian kernels are generally known to perform well in nonlinear classification. However, Gaussian kernels also add another degree of freedom to optimize over: the width parameter $\sigma$. This is yet another parameter which may have to be tuned by the operator depending on the target anatomy.

For each scan line, a set of features may be generated based on the statistical information of the received RF data and envelopes. In order to help mitigate corruption due to isolated strong scatterers, statistics based on the log of the envelope may also be computed. The identity and uniqueness of the classifier to the problem is the combination of the feature set used to build the classifier. A complete list of features is given below. It is appreciated that for a particular embodiment, any combination of these features may be used for the SVM to identify the target anatomy: (1) skewness of the received RF, (2) mean of the envelope, (3) variance of the envelope, (4), skewness of the envelope, (5) kurtosis of the envelope, (6) mean of the log of the envelope, (7) variance of the log of the envelope, (8) skewness of the log of the envelope, (9) kurtosis of the log of the envelope.

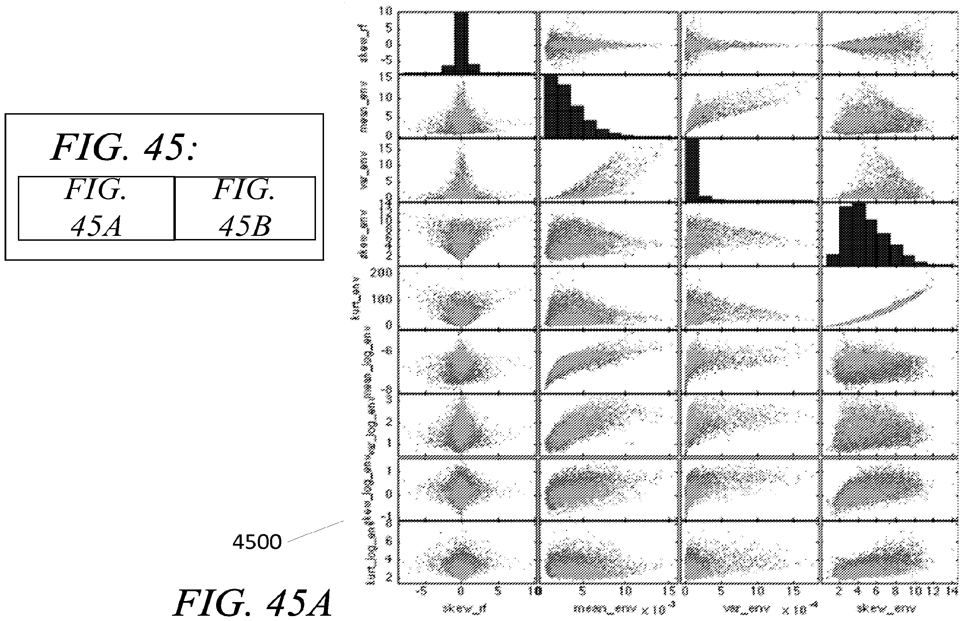

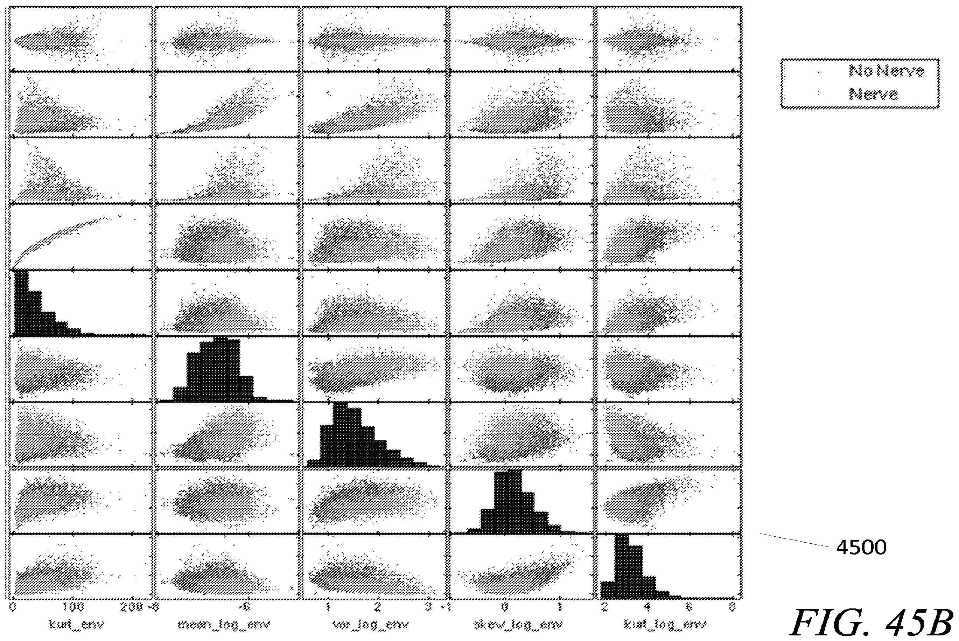

Figure 45 illustrates a plot matrix 4500 demonstrating that the above-recited features do not appear to completely discriminate scan lines containing the nerve from scan lines that do not. Most of the data does not appear to follow a simple linear decision boundary, so a Gaussian kernel with $\sigma = 1$ was used to help preserve nonlinear tendencies. The box constraint parameter was also set to 1 during training.

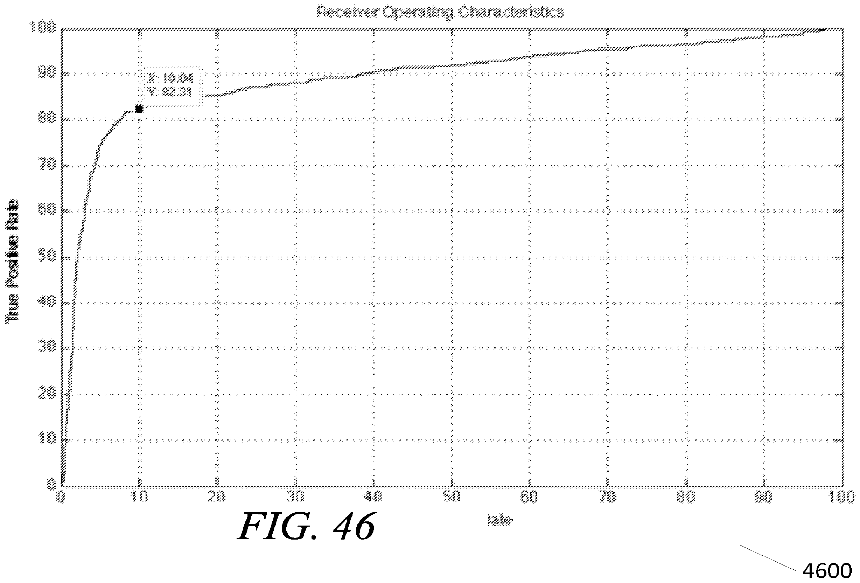

To evaluate the performance of the SVM, an evaluation metric may be computed by a specially-programmed computing device. In some aspects, the receiver operating characteristic may be used as an evaluation metric. For example, as a classification algorithm, the ROC curve plots the true positive rate vs. false positive rate, thus displaying the different trade-offs between operating at various threshold levels. Figure 46 illustrates the results 4600 of the ROC plot, indicating that the classification algorithm appears to have good performance based on the training data distributions.

The testing demonstrated that a Gaussian-based SVM can be a powerful tool in determining the presence of the sciatic nerve in a single scan line. The majority of the power of any multivariate algorithm lies within the features used to describe the data. Utilizing just a single scan line, the system may be able to achieve a true positive rate of over 80% and a false negative rate of less than 10%.

Additional techniques contemplated include post-processing schemes, such as time gain compensation, to accentuate deeper features in the tissue, or using filters such as median filters to remove some of the strong peaks from the energy signals.

The detection of the nerve (or any other anatomical feature) may be automated. Once the anatomical feature is detected, an audio or visual signal such as "beeping" sound or a flashing light signal (or similar signal) may be given to a physician to indicate that they, or the device, are within a certain distance from the nerve.

Automatic detection of nerve may be based on single scan lines, and may compare the b-mode scan lines captured by the probe with the known scan lines of the target anatomy. In some aspects, the detection system may notify the operator that the captured scan lines are identical to, or are within a certain predetermined value of, the known scan lines of the target anatomy (e.g., the known scan lines of the target anatomy may represent a unique signature). The detection system may also be calibrated to determine the proximity of the tip of the probe to the target anatomy and notify the operator when the tip of the probe is within a set distance (e.g. 1 mm). Furthermore, the system will be configured to notify the operator the spatial location of the target anatomy and inversely the spatial location of non-target anatomy.

Further Details Regarding Aspects of the Present Disclosure

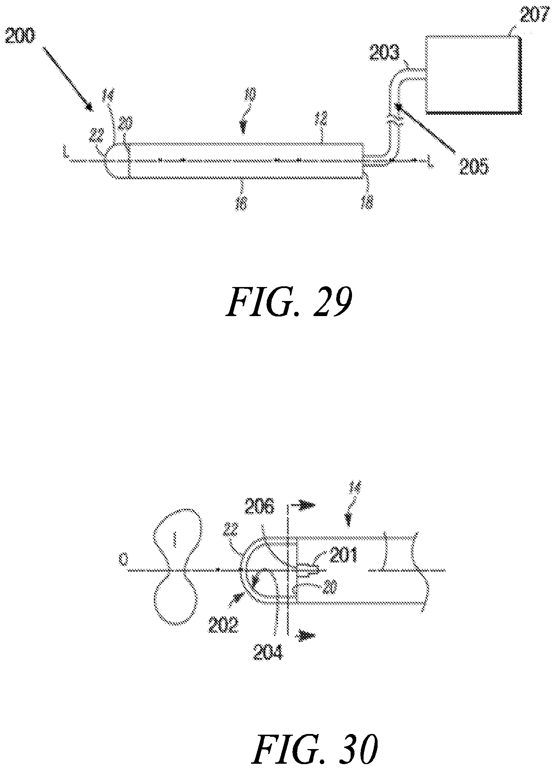

In some aspects, the device 10 may be equipped with an image capture system 200 as shown in Figures 29 and 30 used to detect certain portions or aspects of the anatomy (e.g., nerve or vessels). The image capture system 200 can be used as a stand alone or a complimentary detection technique to the ultrasound imager 24. In some aspects, the tip 22 in this embodiment is a lens 23 that has an outer surface 202 and an inner surface 204. It is appreciated that the outer surface 202 of the lens 23 need not be the same shape of the inner surface 204 and may differ depending on the desired optical performance. The lens 23 may or may not provide a magnification of an image, I, beyond the lens 23. In this embodiment, the lens 23 provides no magnification. The lens 23 in this embodiment is clear, but can also be tinted or provided with a color filter as will be discussed below and may have anti-fog, anti-condensation, and/or anti-reflection coatings or properties.

As further seen in Figure 30, the distal portion 14 of the device 10 further includes the image capture system 200 having an image capture device 201. The image capture device 201 may include an image capture sensor 206 that is disposed adjacent to the distal end 20 of the main body 16. The image capture device 201 can be connected to a flexible sheath 203 that may carry a fiber optic cable 205 that connects the image capture device 201 to a housing 207 that houses the image processing components. It is also contemplated that the image capture device 201 can be wirelessly coupled to the image processing systems.

An image capture output 208 may be included that is in communication with an image control system that can adjust the properties of the image quality. It can also be appreciated that the image capture device 201 may wirelessly transmit images and video to the image control system without any hard wire components. The term "image capture device" may include devices configured to record or capture either still or video images, such as video cameras, digital cameras, CCD sensors, and the like. One example of an image capture device that may be used with the device may be a 1/18" CMOS camera with OMNIVISION sensor OV 6930. However, those skilled in the art will easily contemplate the settings & components such as the "image capture device," illumination device, and the like in accordance with the present disclosure as described herein.

In one aspect, the image sensor 206 of the image capture device 201 may be disposed within the distal portion 14 of the main body 10. The image sensor 206 or the capture device 201 may be at the most distal end 20 of the main body 16 such that it forms a distal surface 210 of the image sensor 206 (or capture device 201) or is flush with the distal end 20 of the main body 16. In some aspects, "image sensor" may be synonymous with "image capture device." In some aspects, the image sensor 206 may be set back proximally from the distal end 20 of the main body 16, depending on the application. For example, the image sensor 206 may be recessed from the distal end 20 of the main body 16. Alternatively, the image sensor 206 or the image capture device 28 may extend from the distal end 20 of the main body 16 towards the inner surface 204 of the tip 22.

The image capture device 201 may define an optical axis O. As shown in Figure 30, the optical axis O is the axis collinear with the longitudinal axis L defined by the main body 16 as discussed above. However, the optical axis O may also be or offset from the longitudinal axis L. The image capture device 201 will have a field of view $\alpha$, varying between 5 and 180° depending on the specific application.

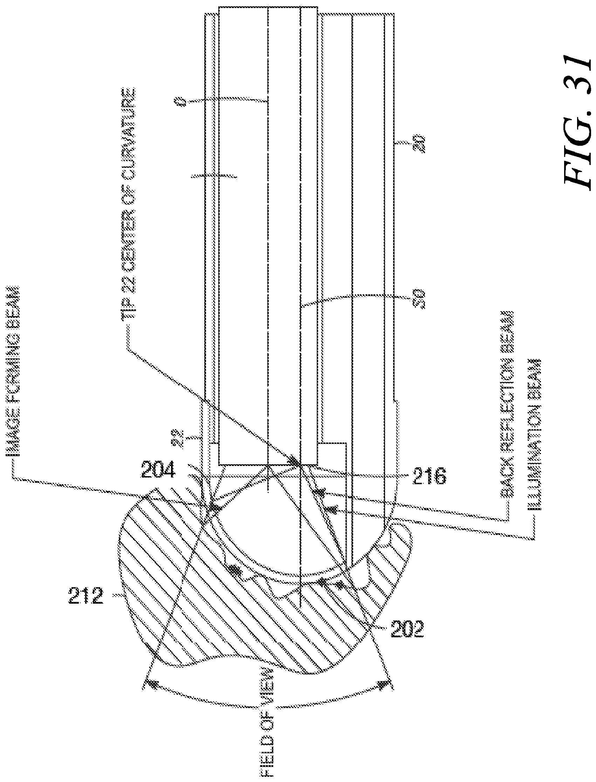

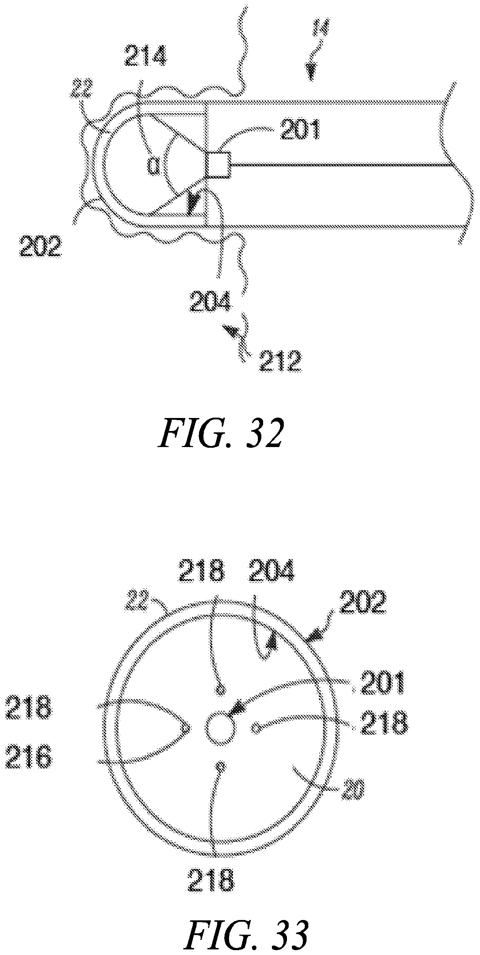

The image capture device 201 may be configured to capture an image I that exists just beyond the outer surface 202 of the tip 22. Specifically, as better shown in Figures 31 and 32, the tip 22 may be configured to dissect the anatomy of a patient, designated here as element 212. As seen here, the tip 22 while dissecting the anatomy may create a working space 214 by way of its shape. This may enable the image capture device 201 to view the anatomy during the dissection process instead of having the anatomy directly abut the image capture device 201 which may distort the image quality.

In this embodiment, the working space 214 may be defined as the space between the distal surface 210 of the image capture device 201 and the outer surface 212 of the tip 22. The working space 214 may permit the image capture device 201 to view a portion of the anatomy that is within its viewing angle $\alpha$. Without the working space 214, the anatomy might abut and thereby obstruct the image capture device 201 thereby preventing an illumination device 216 from illuminating the anatomy and the image capture device 201 from capturing an image. The working space 214 in some aspects may be primarily filled with air, but it can be appreciated that the working space may be filled with other materials, such as a liquid or other gases. In the alternative, the working space 214 may be created by a tip that is solid such that the inner surface 204 of the tip 22 is adjacent to the distal end 20 of the main body. It can be appreciated that the working space 214 may create a distance between the outer surface 202 of the tip 22 and the image capture device 28 of 2 mm to 10 mm or greater.

The distal portion 14 of the main body 16 may also include the illumination device 216, as shown in Figure 33, which is a cross sectional view of Figure 30. The illumination device 216 may include a set of light emitting diodes 42 ("LED"). It can be appreciated that the number of LEDs 218 and the location of each LED 218 with respect to the image capture device 201 may vary. For the example, there may only be one LED 218 for a particular application. Conversely, there maybe as many as four or more LEDs 218. In some aspects, the LEDs 218 are spaced equidistant from one other, but it is not a requirement and the LEDs 218 may not be equally spaced. Other illumination devices 216 may include a light source such as near-infrared LED, mid-infrared LED, other LEDs of various wavelengths ranging from UV to infrared, or any similar light source known in the art. The illumination device 216 may also take the form of a light source emitting from an annular ring around the image capture device 201 or a plurality of light fibers angularly spaced around the image capture device 201.

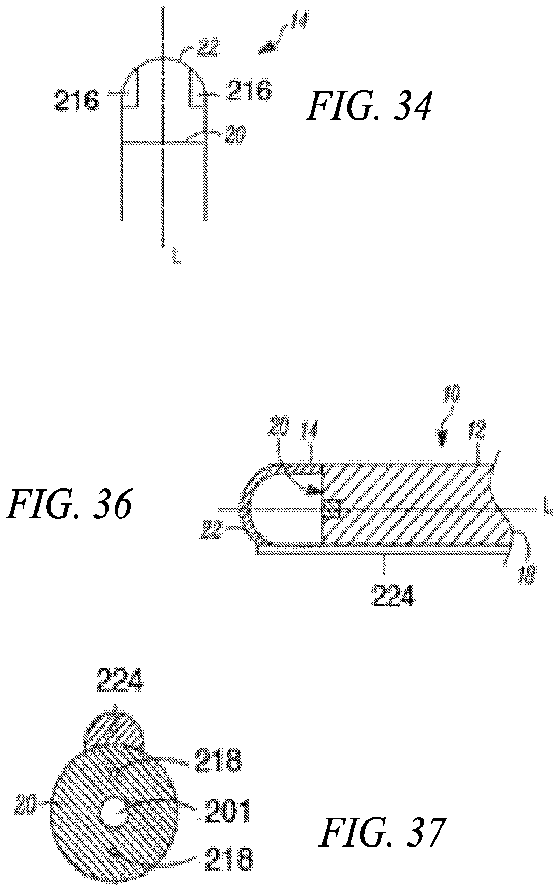

The illumination device 40 in the device 10 may be disposed within the distal end 20 of the main body 16. However, the illumination device 216 may be external to distal end 20 or tip 22 or be embedded in the tip 22 such that the illumination device 216 does not create reflection on the inner surface 204 of the tip 22, as a reflection may impair the field of visibility of the image capture device 201. For example, and without limitation, the illumination device 216 may be disposed on either side of the longitudinal axis L of the main body 16 as shown in Figure 34 and distal from the distal end 20 of the main body 16.

Regardless of the type of illumination device 216, the intensity of the illumination device 216 may be adjusted to as to change the level of illumination of the anatomy of a patient. Moreover, if the illumination device 216 includes more than one illumination sources, certain of the illumination sources may be turned off while others remain on, and that the intensity of each source may be independently adjusted.

The illumination device 216 may also include color filters to as to highlight certain features of the patient's anatomy such as a nerve or vessel, through a filter. In some aspects where the illumination device 216 consists of one or more LEDs 218, different color LEDs, such as red blue, yellow, green or others, may be used to enhance or highlight certain features of a patient's anatomy. One alternative to placing the filters with the illumination device 216 may be to include the filters with image capture device 201. The filters may include color filters, including red, orange, yellow, green, magenta, blue, violet, and the like. The filters may also include band pass-filters, such as UV, IR, or UV-IR filters to better view the desired anatomy. Alternatively, or in conjunction with the above, the illumination device 216 may rely on ultraviolet to detect certain features of the patient's anatomy, such as a nerve. In this embodiment, the illumination device 216 may include a light source irradiating the desired portion of a patient's anatomy with illumination light including excitation light of a wavelength band in an ultraviolet or visible region. It is also contemplated that the illumination device 216 may have both illumination means for emitting illumination light in the visible and ultraviolet regions simultaneously and where the image capture device having an appropriate spectral response can capture images in both regions.

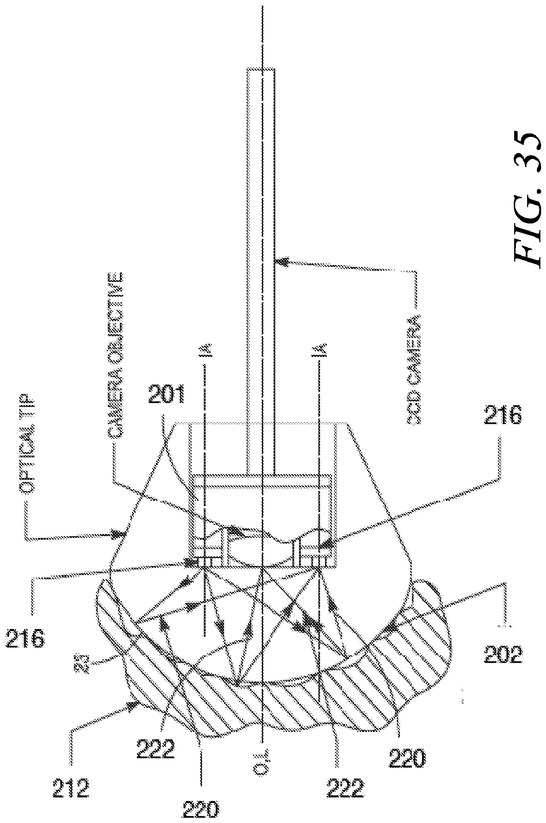

The illumination device 216 may have an illumination axis IA as disclosed in Figure 35. The illumination axis IA may or may not be collinear with the optical axis O or the longitudinal axis L of the device. The illumination device 216 and location of the illumination axis IA may result in a reflection captured by the image capture device 201 that may distort the image of the patient's anatomy. The reflection may be caused by the rays generated by the illumination device 216 reflecting from an outer surface 202 or inner surface 204 of the tip 22 or the lens 23. To minimize and/or eliminate the reflection caused by the illumination device 216, the illumination axis IA may be displaced relative to the optical axis O of the image capture device 201. In the aspect as shown in Fig. 35, the optical tip 22 is shown as a solid lens with the elliptical outside surface 202. One property of elliptical surfaces is that if a light source is placed at its focus all light rays on the surface of the ellipse are reflected to the second focus. Each of two light sources 216 in the form of LED or optical fiber may be placed at the focal point of elliptical surface 202. The illumination rays 220 reflected from elliptical surface may concentrate at foci of this surface while the rays 222 reflected from the tissue abutting the surface 202 may be directed toward the capture device 201. This arrangement eliminates the direct optical reflection of illumination beams into the image capture device 201 or sensor 206 while maintaining its on-axis position. The offset between the optical axis O and the illumination axis IA may vary in range including between 1 to 2.5 mm.

Another embodiment of the device that includes an offset between the optical axis O and illumination axis IA is shown in Figure 31. As shown in this embodiment, the tip optical axis SO of the tip 22 is offset from the optical axis O of the image capture device 201 to reduce or minimize the amount of reflection captured by the image capture device 201. In this embodiment, the illumination is provided by the annular ring of optical fiber around the lens of the image capture device 201. The illumination beams reflected from the inner surface 204 will focus on the tip 22 center of curvature. Because the tip optical axis is offset in relation to the optical axis of the image capture device 201 its center of curvature is outside of the objective field of view and therefore reflected beams do not degrade the image quality.

The image capture system 200 may be used with the glove embodiment discussed below where the image capture device 201, its tip 22, sensor 206, illumination device 216 may be placed on a distal portion 114 of the index finger 112 of the glove 110 as described below. The image capture system 200 may also be used independent, or in conjunction, with the ultrasound imager 24 as described above in the device 10 or glove 110 embodiments.

As shown in Figures 36-37, the device 10 may also include a conduit 224 disposed at least partially within the main body 16 and the tip 22. In the aspect shown in Figure 36, the conduit 224 may be off-set from the longitudinal axis L of the main body 16. However, in some aspects, the conduit 224 may be collinear with the longitudinal axis L of the main body 12. The conduit 224 may extend from the proximal end 18 of the main body 12, or may be formed along only a portion of the main body 12 and extend all the way through the distal portion 14 of the device, including the tip 22. The conduit 224 may be used in the ultrasound or visualization embodiments of this device. The conduct 224 may also be placed in the along the longitudinal axis of the main body 16, which would require the transducer 26 to be offset to accommodate the conduit 224.

Stated differently, the location of the conduit 224 may vary and is application dependent. For example, and without limitation, the conduit may be placed closer to the longitudinal axis L than shown in the embodiment disclosed in Figure 36. In the alternative, the conduit may be placed on the exterior of main body 16 of the device to as to form a raised portion that extends along a direction that is substantially parallel to the longitudinal axis L of the main body 16. In this embodiment, the conduit 224 would reside in the raised portion as shown in Figure 37, which is a cross-sectional view of the distal end 20 of the main body 16 of this embodiment.

In addition, it is contemplated that there may be more than one conduit 224 for a particular device 10 thereby allowing an operator to simultaneously place multiple instruments into the patient's anatomy. The diameter of the conduit is application dependent by can vary and may be between .3 mm to 5.5 mm.

A conduit 224 as shown in Figures 36-37, may be configured to receive a number of instruments, including a guide wire to guide the device 10 through the anatomy of a patient, a k-wire to anchor the device 10 to a surgical site such as the disk space between two vertebra, an illumination device, such as an optical fiber, to provide additional illumination to a particular region of the patient's anatomy, an ultrasound probe to image or detect particular regions of a patient's anatomy, such as the one described above, a fiber optic cord that emits visible or infrared light to image or detect particular regions of a patient's anatomy, a nerve stimulator for neuromonitoring, and the like.

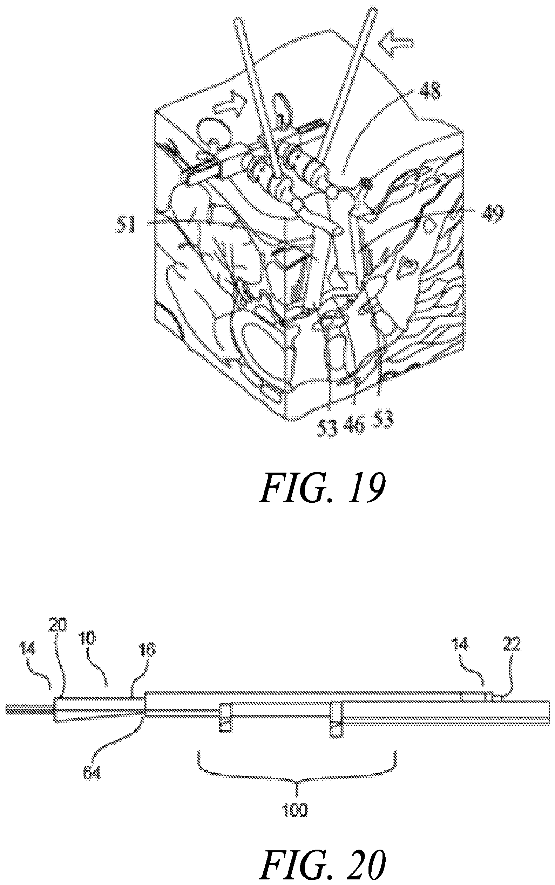

Various aspects of the present disclosure are contemplated for being used in connection with minimally invasive surgery (MIS). The device 10 may be used for a variety of MIS procedures, including but not limited to, a lateral retroperitoneal interbody fusion (LLIF) (e.g., XLIF, DLIF), Axial Lumbar Interbody Fusion (AxiaLif), Transforaminal Lumbar Interbody Fusion (TLIF), Posterior Lumbar Interbody Fusion (PLIF), Anterior Lumbar Interbody Fusion, Trans-thoracic lumbar interbody fusion, Retropleural Thoracic Fusion, Interbody Fusion utilizing Kambin's Triangle, and Cervical/Thoracic/Lumbar Laminectomies, Foraminotomies and Diskectomies. The device 10 may be used to confirm that the area is clear of other anatomical parts, such as blood vessels, abdominal/pelvic viscera, nerve roots, and spinal cord. As shown in Figure 19, once at the surgical site 46, the device 10 may be used to illuminate the surgical site 46, to allow a surgeon to introduce instruments (e.g. K-wire) to the surgical site via a conduit formed within the main body 16 of the device 10 or allow a retractor system or dilator system to create direct visualization and a working portal of the surgical site without the device 10.

As described above, there can be a number of applications for which this device 10 may be used, which require the similar steps to access the surgical site. The method of use described below is in connection with performing an LLIF, but it can be appreciated that the device 10 can be used in a similar fashion for performing other MIS procedures as mentioned above.

In operation, the ultrasound imager 24 is used to detect the patient's anatomy as described herein. A surgeon may rely on the image or audio queues generated by the ultrasound imager 24 to detect the presence (or absence) of a nerve thereby allowing the surgery to reposition (or continue advancing) the device 10 through the patient's anatomy towards the surgical site 46. The ultrasound imager 24 may also be used to confirm the image captured by an image capture device (not shown) is accurate by confirming the presence or absence of a targeted anatomical feature (e.g. nerve). The image capture device may consist of a camera or the like disposed within the distal portion 14 of the device 14 so as to capture an image of the region distal to the device 10.

The image capture system 200 may also be used in a similar fashion to visually detect the patient's anatomy. The image capture system 200 may be used to confirm what is detected by the ultrasound imager 24, or may be used independently to detect certain portions of the patient's anatomy.

Once the muscles are split and the surgical site 46 is reached, the surgeon can place a k-wire through the conduit to confirm that the surgical site 46 is reached and anchor the device 10 with respect to the surgical site 46. A retractor tool 48 is put into place to give the surgeon a direct surgical working conduit to the surgical site 46. Alternatively, a series of dilators may be sequentially placed over the main body 16 to create the working space. Once this direct access to the spine is achieved, the surgeon is able to perform a standard discectomy (removing the intervertebral disc), corpectomy (removing the vertebral bone) or fusion (uniting two bones together) with surgical tools.

An embodiment of the retractor system 48 may include a first blade 49 and a second blade 51, both of which are semi-circular in shape that form an opening that fits snugly around the outer diameter the main body 16. It is appreciated that the cross-sectional shape of the blades can mimic the shape of the main body 16 (e.g., triangular, oval, square, rectangular, etc.). Once at the surgical site, the retractor blades 49, 51 are configured to separate relative to one another so as to expand the dissection and to enable the device 10 to be removed and allow for direct visualization of the surgical site 46 as shown in Figure 19. It is contemplated that the distal ends 53 of the first 49 and second 51 blades are adjacent to the distal portion 14 of the main body 12. Any known type retractor system may be used with the device 10.

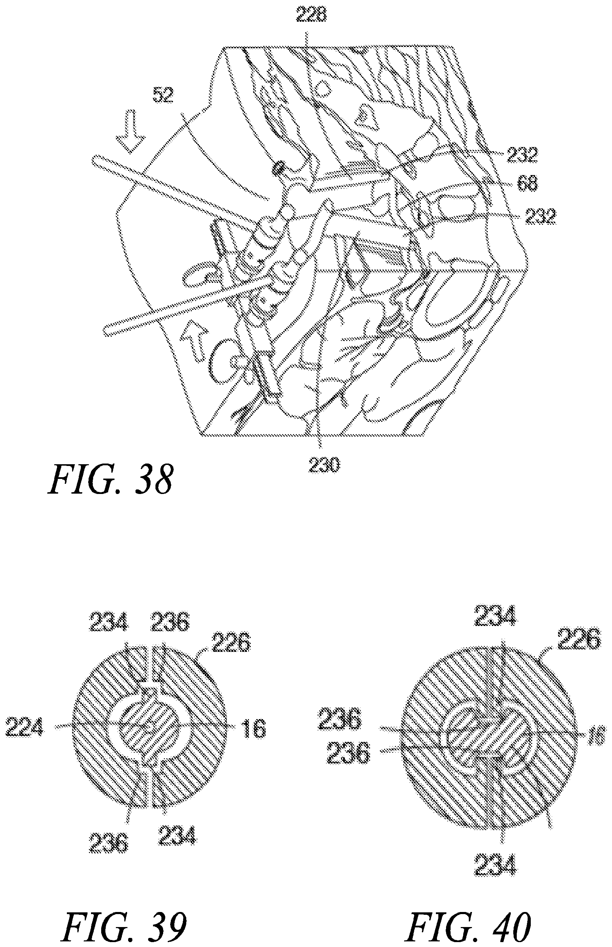

In one embodiment, a retractor system 226, like the one disclosed in Figure 37, may be disposed over the device 10 and configured to expand to create a working space within the anatomy of the patient once a surgical site, or other location where a surgical procedure is to take place, is reached. This embodiment of the retractor system 226 includes a first blade 228 and a second blade 230, both of which are semi-circular in shape that form an opening that fits snugly around the outer diameter the main body 16. Once at the surgical site, the retractor blades 228, 230 are configured to separate relative to one another to expand the dissection so as to enable the device 10 to be removed and allow for direct visualization of the surgical site 68 as shown in Figure 38. The distal ends 232 of the first 228 and second 230 blades may be adjacent to the distal portion 14 of the main body 12. Any type known retractor system may be used with the device 10. It can be appreciated that stimulation electrodes, visualization cameras and illumination devices (optical, ultrasound, infrared and ultraviolet) may also be placed along or within the retractor blades 228, 239 so as to allow for nerve detection as discussed above.

As shown in Figure 39, a cross-sectional view of the retractor 226 disposed around the device 10, the main body 16 of the device 10 may have a raised channel or channels 234 disposed along its length in a direction along the its longitudinal axis that is configured to slidingly receive a complimentary groove 236 formed by the first and second blades 228, 230 of the retractor system 226. In some aspects, the raised channel 234 and/or groove 236 may have a square, rectangle, semi-spherical or a similar cross- sectional shape. Further, the number and location of the channel 234 and groove 236 may vary. For example, but without limitation, the may only be one channel/groove running along the main body 16.

Alternatively, there may be more than two channels/grooves that are equidistantly placed about the outer surface of the main body 16. The channel and groove may also be transposed, such that the grove 236 is on the main body 16 and the raised channel 234 is formed along the blades 228, 230, as shown in Figure 40, such that the groove 236 prevents the blades 228, 230 from expanding when the retractor 226 is disposed over the device 10. The channel 236 and grove 72 may extend along only a portion of the main body 16 of the device 10 and the blades 228, 230.

The retractor system 226 may consistent of multiple sets of blades 228, 230 with varying thickness and diameter. For example, and without limitation, the blades 228, 230 may vary in size so as to have an overall outer diameter ranging from 2 mm to 80 mm when in the closed (e.g., collapsed) configuration. Further, the blades 228, 230 may be configured such that they create a 2 mm to 220 mm opening within the patient's body when in the expanded configuration. The device 10 and retractor system 226 may be configured such that a first set of blades 228, 230 having a larger retracted diameter may be used to create a first opening within the patient's body and then a second set smaller diameter blades having a retracted diameter that is different than the first set of blades 228, 230 may be slidingly disposed over the main body 16 and within the first opening and retracted to open a second opening within the patient's body at a location distal to the first opening. The openings created by the first and second set of blades may have different opening diameters. The retractor system 226 may allow the operator to create multiple openings having different retracted diameters at different anatomic levels within the patient. A light source (not shown) may be disposed at the distal ends, or along the length, of the blades 228, 230 to illuminate the opening within the patient's body and illuminate the region of the patient's anatomy within and distal to the opening created by the blades 228, 230 In addition, a conduit (not shown) may also be formed within the blades so as to receive one or more of: a medical instrument, such as a k-wire to anchor the blades 228, 230 to a surgical site such as the disk space between two vertebra; an illumination device, such as an optical fiber, to provide additional illumination to a particular region of the patient's anatomy; an electrical conduit to provide neural stimulation; a ultrasound probe to image or detect particular regions of a patient's anatomy; a image capture device; a fiber optic cord that emits visible or infrared light to image or detect particular regions of a patient's anatomy; and the like. The blades 228, 230 may be made out of any preferable surgical grade material, including but not limited to, medical grade polymer including PEEK (polyether ether ketone), and may be transparent (e.g. made out of clear plastic) or translucent.

In another embodiment, the retractor system 226 may be integrated with the device 10 such that it forms part of the main body 16 and can be deployed once at the surgical side. The retractor system 226 in this embodiment will expand radially away from the longitudinal axis of the main body 16 to expand the path created by the main body 16. The main body 16 can then be withdrawn from the surgical site so as to create a working portal within the retractor system 226.

A series of dilating cannulas (e.g. dilators 100), as shown in Figure 20, can also be slidingly placed around the main body 16 of the device 10 so as to expand the diameter of the dissection made by the distal portion 14 of the device 10. The technique of employing a series of dilating cannulas to create a working space for direct visualization used in other medical procedures to create a working space can also be used in conjunction with the device 10.

After the disc material is removed, the surgeon may be able to insert the implant through the same incision from the side. This spacer (cage) may help hold the vertebrae in the proper position to make sure that the disc height (space between adjacent vertebral bodies) is correct and to make sure the spine is properly aligned. This spacer together with the bone graft may be designed to set up an optimal environment to allow the spine to fuse at that particular segment. The surgeon may use fluoroscopy to make sure that the spacer is in the right position. The surgeon may then remove the refractor and suture the incisions.

In the alternative, when the tip 22 is disposed adjacent to the psoas muscle, the surgeon may slide a first set of blades of the retractor system over the device and expand the retractor system 48 to create a first working space (also referred to as a superficial dock). This working space may allow the surgeon to visually inspect the psoas muscle and the surrounding region either via naked (eye) inspection or with the optical camera/dissector (e.g., one or more components of device 10). Next, the surgeon may continue the procedure by using the device, which is now disposed within the first working space to dissect through the psoas muscle as described herein. Once the tip 22 has reached the surgical site, which is the disc space here, a second set of retractor blades which are smaller than the first set of blades are slid over the device 10 and expanded to create a second working space that is smaller in diameter than the first working space. The surgeon will then continue with the procedure in the manner discussed herein. One benefit of establishing the first working space may be that it allows the surgeon to remove the device 10 from the surgical site once the procedure is completed at the first surgical site and reposition and reinsert the distal tip 22 of the device 10 within the first working space that is formed above the psoas muscle at a second location to allow the surgeon to penetrate the psoas muscle to reach a second surgical site to conduct and complete another procedure or a multi-level procedure in which psoas dissection is currently dangerous because o the interposed neurovascular structures (L3-4 and L4-5 disc space or a lumbar corpectomy - removal of two discs and the intervening bone). It is appreciated that the tip 22 is optional and the distal end 21 of the device 10 maybe the portion of the device that is advanced towards the surgical site.

The device 10 may also be used for performing an axial lumbar interbody fusion (AxiaLIF). At surgery, the patient may be positioned prone with maintenance of lordosis and the legs spread. A catheter is inserted into the rectum will allow air to be injected during the procedure for visualization of the rectum. After the surgeon makes a small incision (15-18 mm) lateral to the tip of the coccyx, the distal tip 22 of the device 10 is inserted through the incision and is passed into the pre-sacral space. The surgeon uses the distal portion 14 of the device 10 to sweep and scan the pre-sacral space to confirm that the space is clear of any offending anatomy (e.g.

colon, rectum). The device 10 is gently passed along the anterior cortex of the sacrum and in the midline to an entry point usually close to the S1-2 junction. Once the trajectory is chosen, a sharp beveled pin is then driven into the L5-S1 interspace, either through the conduit 36 or after the retractor system 48 is deployed. The retractor system, 48 or a series of dilators is used to create approximately a 10 mm opening into the sacrum through which a 10 mm channel is drilled into the L5-S1 disc. The device 10 is then withdrawn from the pre-sacral space and the surgeon then performs the remaining steps of the AxiaLIF procedure.

The device 10 may also be used to allow direct access to Kambin's triangle (ExtraForaminal Lumbar Interbody Fusion). For this procedure, patients are placed in the prone position typically onto a Jackson Table using a radiolucent frame that allows for restoration of lumbar lordosis. Fluoroscopic imaging is utilized to identify the epiphyseal plate of the upper and lower vertebral body by controlling the cranial-caudal angle of the image intensifier. Additionally, the fluoroscopic image is rotated by 20-35 degrees toward the region, so that the superior articular process can be seen at the middle of the intervertebral disc. At this location, the tip 22 of the device 10 can be inserted percutaneously targeting the area commonly referred to as Kambin's triangle. Kambin's triangle is defined as the area over the dorsolateral disc. The hypotenuse is the exiting nerve root, the base (width) is the superior border of the caudal vertebra and the height is the dura/traversing nerve root.

The device 10 may also be used to ultrasonically identify various anatomical features such as the exiting root, radicular artery, thecal sac and the disc space. A k-wire can then be place into the disc space via the conduit 36 under ultrasonic detection via the device 10 allowing for docking of the dissector/retractor system 48. Subsequent dilation can then be performed allowing for access in the intervertebral foramen while directly visualizing neurovascular structures using the device and avoiding these structures when identified by the surgeon.

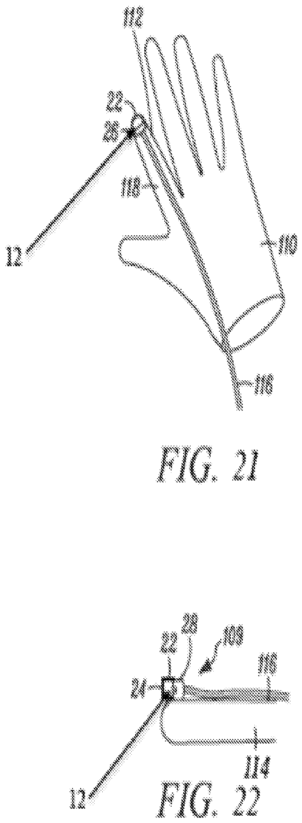

In another embodiment, shown in Figure 21, an ultrasound imager 24 may be used in conjunction with a glove 110. In this embodiment, the operator may rely on tactile feedback provided by touch while still enabling ultrasonic imagining/scanning of a patient's anatomy. More specifically, the glove system (or device) may allow for tactile feedback that facilitates the dissection and separation of tissue namely neurological, vascular and peritoneal structures. In general, tactile feedback allows for dissection of tissue in normal surgical procedures without the unique perspective of direct visualization that may not be permissible in some minimally invasive/percutaneous techniques.

The ultrasound imager 24 may include a transducer 26 that is configured to emit sound waves may be disposed at the distal end 20 of the glove 110. In one embodiment, the transducer 26 is located along a distal portion 114 of the index finger 112 of the glove 110. As better shown in Figure 22, a tip 22 forms part of, or is connected to, the distal portion 114 of the index finger 112 such that the outer surface 24 of the tip 22 does not extend beyond the very most distal part of the index finger 112. Of course, it is appreciated that the tip 22 may extend beyond the distal portion depending on the embodiment.

Connected to the transducer 26 flexible conduit 116 that may carry a cable that connects the transducer 26 to a housing that contains the remaining portion of the ultrasound imager 24. The flexible conduit 116 runs along the length of the index finger 112 and a top portion 118 of the glove 110. However, it can be appreciated that the conduit 116 can run along any length or surface of the glove 110 and is application dependent. The flexible conduit 116 may also provide a channel to carry a k-wire or other instrument that can be slidingly disposed within the flexible conduit 116 (as will be further discussed below). The flexible conduit 116 runs through and is in communication with a unit 109 such that a portion of the flexible conduct 116 provides an opening 120 in the unit 109 at the distal portion 114 of the index finger 114.

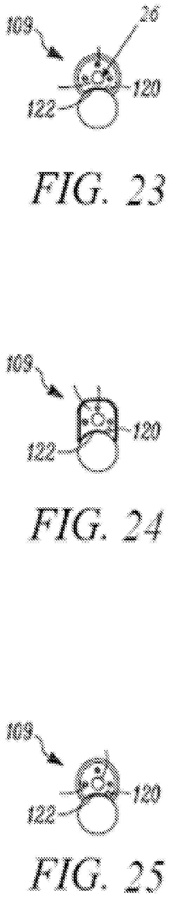

The unit 109 may have a bottom portion 122, as shown in Figure 23. The bottom portion 122 may have a concave curvature so as to provide a complimentary fit once an operator's hand is placed within the glove 109. In addition, a proximal portion 124 of the unit 109 may have a taper so as to cause minimal disruption to a patient's anatomy as the unit 109 is articulated during a procedure. Further, the unit 109 may have an overall semi- circular or cylindrical shape or the like so as to minimize any inadvertent disruption to the patient's anatomy during a procedure and to maintain a small overall profile as shown in Figures 24 and 25. For example, the height of the unit 109 may be less than the overall width to as to achieve a low profile. Alternatively, the outer portion of the unit 109 may not extend beyond and become collinear with the width of the index finger 112 to maintain a low profile. The external outer diameter of the unit 109 may range from .5 to 20 mm and outside of this range depending on the desired application. The length of the unit 109 can range from .5 to 10 mm but also may fall outside of this range depending on the application. It is appreciated that more than one transducer 26 may be positioned along the distal portion of a finger such that they provide side facing scans to generate a multi-directional (e.g. 180°- 300°) scan of the patient's anatomy.

Transducers 26 may be side positioned (e.g. on either side of the index finger 112) so as to provide for multi-directional scanning of the patient's anatomy to detect the nerve or target anatomy. The side positioned transducers may be configured to scan the anatomy around in a circumferential direction around the index finger 112 to detect the nerve (or other target anatomy) not detected by the transducer positioned at the distal end of the main body 16. The multi-directional scanning may enable the system to generate a scan image of the patient's anatomy in multiple directions as the index finger 112 of the glove 110 is advanced through the patient's anatomy. As discussed above, the system that is in communication with the transducers can then detect the nerve even that is not captured by the forward scanning transducer.

The glove embodiment can be used in connection with minimally invasive surgery (MIS). The glove 110 may be used for a variety of MIS procedures, including but not limited to, Lateral Retroperitoneal Interbody Fusion (LLIF (e.g., eXtreme Lateral Lumbar Interbody Fusion (XLIF), Direct Lateral Interbody Fusion (DLIF)), Axial Lumbar Interbody Fusion (AxiaLif), Transforaminal Lumbar Interbody Fusion (TLIF), Posterior Lumbar Interbody Fusion (PLIF), Anterior Lumbar Interbody Fusion, Transthoracic lumbar interbody fusion, Retropleural Thoracic Fusion, Interbody Fusion utilizing Kambin's Triangle, and Cervical/Thoracic/Lumbar Laminectomies, Foraminotomies and Diskectomies. The glove 110 may be used to confirm that the area is clear of other anatomical parts, such as blood vessels, abdominal/pelvic viscera, nerve roots, and spinal cord.

As described above, there can be a number of applications for which this glove 110 may be used, which require the similar steps to access the surgical site. The surgeon may rely on the image or audio queues generated by the ultrasound imager 24 to detect the presence (or absence) of a nerve thereby allowing the surgery to reposition (or continue advancing) the glove 110 through the patient's anatomy towards the surgical site 48.

Once the muscles are split and the surgical site 48 is reached, the surgeon can place a k-wire through the conduit to confirm that the surgical site 48 is reached and anchor the glove 110 with respect to the surgical site 48. A retractor tool is put into place to give the surgeon a direct surgical working conduit to the surgical site 48. Alternatively, a series of dilators may be sequentially placed over the k-wire to create the working space. Once this direct access to the spine is achieved, the surgeon is able to perform a standard discectomy (removing the intervertebral disc), corpectomy (removing the vertebral bone) or fusion (uniting two bones together) with surgical tools.

After the disc material is removed, the surgeon is able to insert the implant through the same incision from the side. This spacer (cage) will help hold the vertebrae in the proper position to make sure that the disc height (space between adjacent vertebral bodies) is correct and to make sure the spine is properly aligned. This spacer together with the bone graft is designed to set up an optimal environment to allow the spine to fuse at that particular segment. The surgeon will use fluoroscopy to make sure that the spacer is in the right position. The surgeon will then remove the refractor and suture the incisions.

The glove system may also be used for performing an axial lumbar interbody fusion (AxiaLIF). At surgery, the patient is positioned prone with maintenance of lordosis and the legs spread. A catheter is inserted into the rectum will allow air to be injected during the procedure for visualization of the rectum. After the surgeon makes a small incision (15-18 mm) lateral to the tip of the coccyx, the distal portion of the index finger 112 and distal tip 22 is inserted through the incision and is passed into the pre-sacral space. The surgeon uses the index finger 112 to sweep and inspect the pre-sacral space to confirm that the space is clear of any offending anatomy (e.g. colon, rectum) visually and by way of ultrasonic imaging. The index finger 112 is advanced along the anterior cortex of the sacrum and in the midline to an entry point usually close to the S1-2 junction. Once the trajectory is chosen, a sharp beveled pin is then driven into the L5-S1 interspace, either through a conduit or after the retractor system is deployed. The retractor system or a series of dilators is used to create approximately a 10 mm opening into the sacrum through which a 10 mm channel is drilled into the L5-S1 disc. The index finger 112 is then withdrawn from the pre-sacral space and the surgeon then performs the remaining steps of the AxiaLIF procedure.

The glove system 110 may also be used to allow direct access to Kambin's triangle (Extraforminal interbody fusion). For this procedure, patients are placed in the prone position typically onto a Jackson Table using a radiolucent frame that allows for restoration of lumbar lordosis. Fluoroscopic imaging is utilized to identify the epiphyseal plate of the upper and lower vertebral body by controlling the cranial-caudal angle of the image intensifier. Additionally, the fluoroscopic image is rotated by 20-35 degrees toward the region, so that the superior articular process can be seen at the middle of the intervertebral disc. At this location, the index finger 112 can be inserted percutaneously targeting the area commonly referred to as Kambin's triangle. Kambin's triangle is defined as the area over the dorsolateral disc. The hypotenuse is the exiting nerve root, the base (width) is the superior border of the caudal vertebra and the height is the dura/traversing nerve root.

The glove system may be used to identify various anatomical features such as the exiting root, radicular artery, thecal sac and the disc space. A k-wire can then be place into the disc space via the conduit under ultrasonic visualization allowing for docking of the dissector/retractor system. Subsequent dilation can then be performed allowing for access in the intervertebral foramen while directly visualizing neurovascular structures using the device and avoiding these structures when identified by the surgeon.

The device 10 may also include infrared technology, which includes an infrared emitting light source and an infrared image capture device. The device 10 would include an infrared radiation detecting elements mounted at the distal portion 14 of the device 10. The infrared array is sensitive at e.g. wavelengths from 2 to 14 micrometers. One embodiment of the infrared aspect of the present disclosure uses a two-dimensional array of microbolometer sensor elements packaged in an integrated vacuum package and co-located with readout electronics on the distal tip of the device 10. It is appreciated that the infrared aspect of this disclosure may be used in conjunction with, or separate from, the other embodiments discussed herein. One such infrared system that could be used with the present disclosure is disclosed in U.S. Patent No. 6,652,452, the entirety of which is incorporated herein by reference.

The device 10 may also utilize Optical Coherence Tomography (hereinafter "OCT") technology as a stand alone detection system or in conjunction with the other embodiments disclosed herein. OCT is an optical signal acquisition and processing method that generates images using near infrared light. By way of background, OCT performs high-resolution, cross-sectional tomographic imaging of the internal microstructure in materials and biologic systems by measuring backscattered or back-reflected light. OCT images are typically two- or three-dimensional data sets that represent the optical back-scattering in a cross-sectional plane through the tissue. Image resolutions of approximately 1 to 15 micrometers may be achieved one to two orders of magnitude higher than conventional ultrasound. Imaging can be performed *in situ* and in real time.

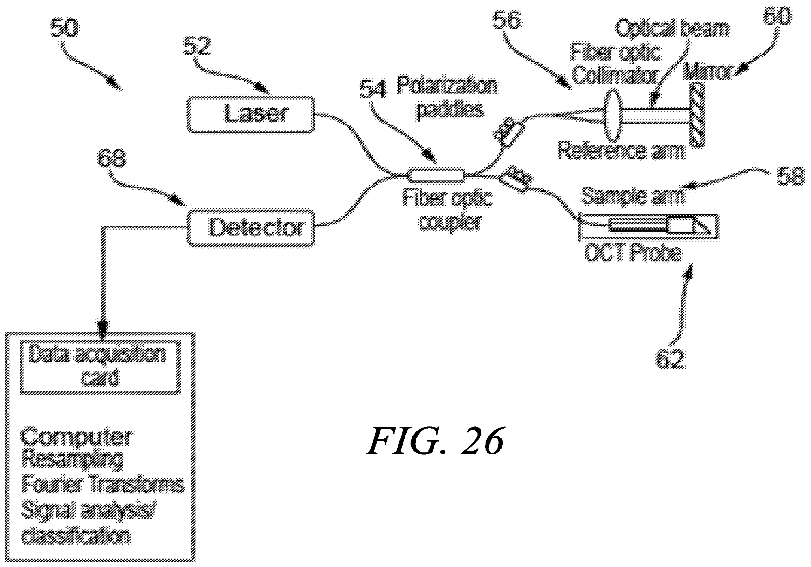

OCT forms depth resolved images by interferometrically detecting the light backscattered from different scatterers within the sample. In a typical OCT system 50, as shown in Figure 26, the light from the laser 52 is split by a fiber optic coupler/beam splitter 54 into two arms i.e. the reference arm 56 and the sample arm 58. The light coupled into the reference arm 56 is reflected back from a fixed mirror 60, while in the sample arm 58 the light is projected through an OCT probe 62, which will be discussed in greater detail below.

The OCT probe 62 is focused onto the sample of interest (e.g. tissue or the anatomy of the patient) through a focusing lens (e.g. a GRIN lens). OCT is a point by point imaging technique where the sample is illuminated by focusing the light from the laser 52 onto a small point (spot size determined by the focusing lens) on the sample. Light in the sample arm 58 travels within the tissue and is backscattered by different scatterers within the tissue and combines with the light from the reference arm 56. If the optical path lengths of the reference 56 and sample 58 arms are matched, an interferogram is formed which can be measured by a photo detector or a spectrometer. The frequency content of the interferogram contains information about the depth and strength of the scatterers that the beam had encountered in the sample. The resulting interferogram is processed to form one-dimensional depth information generally known as an A-scan (a single A-scan would be a single column in the image). The optical beam is then scanned over the sample to generate two- or three-dimensional images. The beam can be scanned using galvanometers in bench-top OCT systems or using MEMS scanners in hand-held OCT devices. This data is sent to and processed by the computer or processor 95.

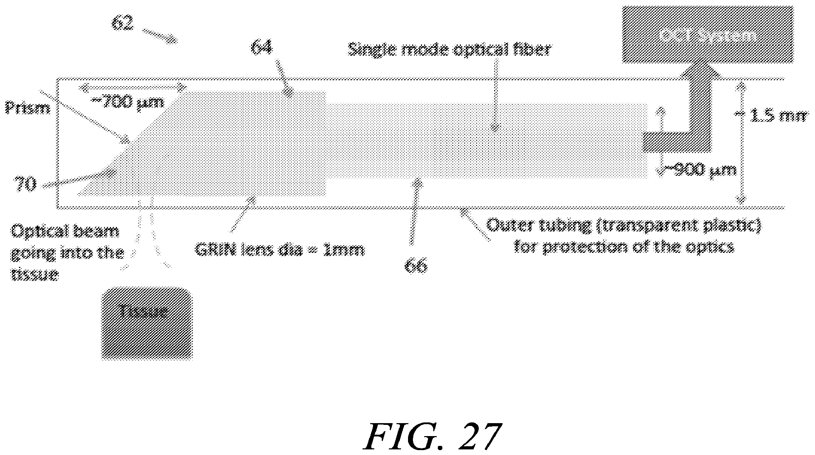

As further disclosed in Figure 27, the OCT probe 62 may include a GRINS lens 64, the diameter of which in this embodiment is 1 mm, but which can vary depending on the intended application. A single mode optical fiber 66 is included in this embodiment that transfers the light rays between the OCT probe 62 and the remaining portion of the OCT system (e.g. the fiber optic coupler 54 or a detector 68). The single mode optical fiber 66 may have a thickness of approximately 900 micrometers and a length of approximately 1.5 m. These specifications, of course, are examples only and can vary depending on the application. Attached to the distal end of the GRINS lens 64 may be a prism 70 for deflecting the light depending on the location and orientation of the target. It can be appreciated that the prism 70 may not be necessary in situations where the surface of the target is directly in front of or substantially perpendicular to the longitudinal axis of the light ray (or beam). In this embodiment, the length of the prism is approximately 700 micrometers, but it is appreciated that the length can vary and is application dependent.

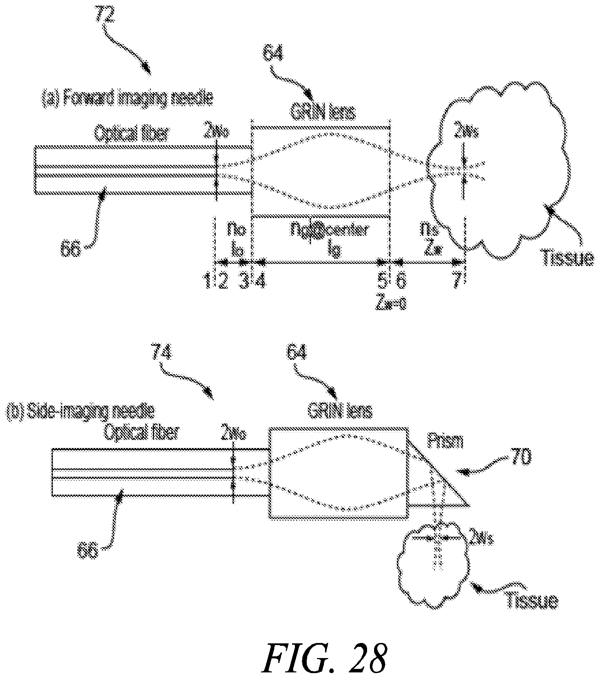

Two different embodiments of the OCT probe 62 are illustrated in Figure 28. The first embodiment is the forward image probe 72, which does not include a prism 70 such that the light ray (or beam) extends outward towards the front of the probe 72 to reach the target (e.g. tissue). The second embodiment 74 contains a prism 70, which allows this embodiment to image targets that are disposed below or at an angle to the tip of the probe 72. The OCT technology may also be incorporated in to the glove system in a manner discussed above with respect to the ultrasound embodiment 110.

Some of the parameters that may be manipulated to optimize OCT imaging include (a) the A-scan rate (the number of A-scans the system can acquire in a second), (b) the axial and transverse resolution, and (c) the imaging depth. The A-line scan rate would determine how fast an OCT system can operate. For a swept source, the OCT system the rate would depend on the wavelength sweeping rate of the laser while for a spectral domain OCT system it is generally limited by the speed of the line scan camera used in the spectrometer. The tradeoff is that at higher A-scan rate the exposure time has to be reduced which can decrease the SNR of the acquired data. The axial resolution (resolution across the depth) is determined by the bandwidth and wavelength of the laser source. In general, higher the bandwidth the better is the axial resolution. The resolution along the transverse dimensions is determined by the numerical aperture of the lens in the sample arm 58. The higher the numerical aperture, higher the transverse resolution, however, the tradeoff is a reduced depth-of-field. Moreover, with an increase in the center wavelength of the source both the axial and transverse resolutions degrade. Finally, the imaging depth is usually limited by how deeply the light can penetrate through the tissue or sample of interest. Higher wavelengths offer greater imaging depth. These and other parameters may be optimized to detect certain features of a patient's anatomy, such as nerve root.

The OCT probe 62 may be positioned at the distal portion 14 of the device 10. Alternatively, the OCT probe 62 may be positioned at the distal end of a k-wire like structure and disposed through the conduit 36. In either embodiment, the OCT probe 62 is configured to image a portion of the patient's anatomy that is adjacent to (or in front of) the distal portion 14 of the device 10. The surgeon may insert the OCT probe 62 to image the patient's anatomy as needed to reach the surgical site. The OCT system 50 may be configured to visually and/or audibly indicate detection of select pre-selected portions of a patient's anatomy (e.g. nerve root). As mentioned above, it can be appreciated that the OCT system can be used independently or in combination with other detection technologies described herein.

In one embodiment, stimulation electrodes may be placed at the distal end of the device 10, such as forming part of the tip 22, or placed at a distal end of an instrument, such as a K-wire, disposed through the conduit 36, to stimulate any nerves in the region adjacent to the distal portion 14 of the device 10. EMG electrodes can be placed on the skin to detect any nerve depolarization in the manner descried in U.S. Patent No. 7,920,922. One manner in which the proximity, location, direction, physiology of the nerve is determined is also disclosed in U.S. Patent No. 7,920,922. It is appreciated that other techniques of detecting nerves using stimulation are known in the art and any of those techniques may be used in conjunction, or integrated, with the device 10 in the manner described above.

The ultrasound imager 24 may be used in conjunction or independent of an image capture device to visualize the patient's anatomy as described herein. Steps and methods describe herein using the ultrasound imager 24 to detect certain features of a patient's anatomy may by supplemented through use of an image capture device. Specifically, the surgeon may rely on the image or audio queues generated by the ultrasound imager 24 to detect the presence (or absence) of a nerve thereby allowing the surgery to reposition (or continue advancing) the device 10 through the patient's anatomy towards the surgical site 48. The ultrasound imager 24 may also be used to confirm the image captured by the image capture device is accurate by confirming the presence or absence of a targeted anatomical feature (e.g. nerve).

Likewise, in operation, the OCT system 50 may be used in conjunction or independent of an image capture device and/or the ultrasound imager 24 to scan and identify the patient's anatomy as described herein and to access the surgical site. Steps and methods used to access the surgical site and avoid target anatomy (e.g. nerve) employing the ultrasound imager 24 may also be performed using the OCT system 50. Furthermore, steps described herein using ultrasound imager 24 may by supplemented through use of the OCT system 50. For example, the surgeon may rely on the image or audio cues generated by the OCT system 50 to detect the presence (or absence) of a nerve thereby allowing the surgery to reposition (or continue advancing) the device 10 through the patient's anatomy towards the surgical site 48. The OCT system 50 may also be used to confirm the image captured by an image capture device is accurate by confirming the presence or absence of a targeted anatomical feature (e.g. nerve).

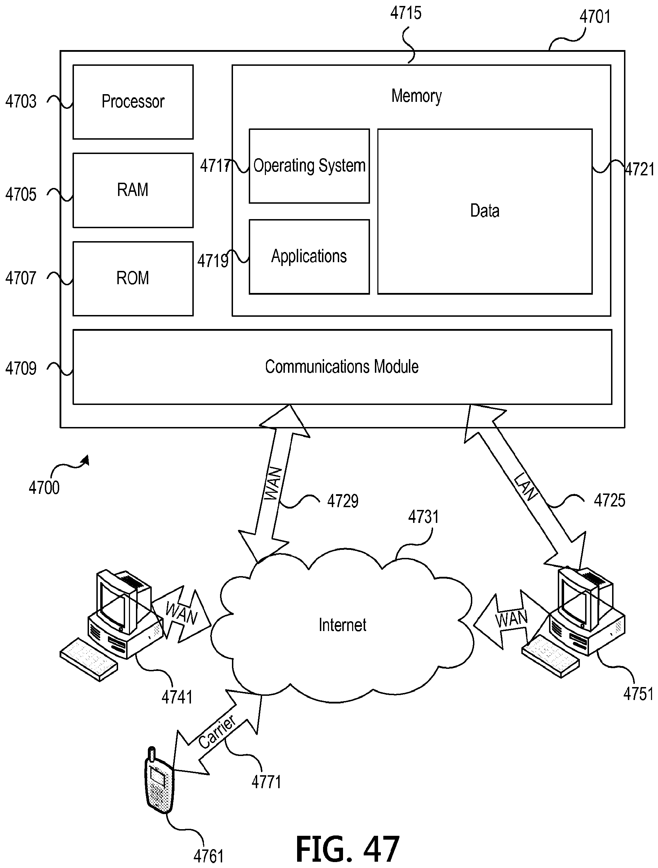

FIG. 47 depicts an illustrative operating environment in which various aspects of the present disclosure may be implemented in accordance with one or more example embodiments. Referring to FIG. 47, computing system environment 4700 may be used according to one or more illustrative embodiments. Computing system environment 4700 is only one example of a suitable computing environment and is not intended to suggest any limitation as to the scope of use or functionality contained in the disclosure. Computing system environment 4700 should not be interpreted as having any dependency or requirement relating to any one or combination of components shown in illustrative computing system environment 4700.

Computing system environment 4700 may include computing device 4701 having processor 4703 for controlling overall operation of computing device 4701 and its associated components, including random-access memory (RAM) 4705, read-only memory (ROM) 4707, communications module 4709, and memory 4715. Computing device 4701 may include a variety of computer readable media. Computer readable media may be any available media that may be accessed by computing device 4701, may be non-transitory, and may include volatile and nonvolatile, removable and non-removable media implemented in any method or technology for storage of information such as computer-readable instructions, object code, data structures, program modules, or other data. Examples of computer readable media may include random access memory (RAM), read only memory (ROM), electronically erasable programmable read only memory (EEPROM), flash memory or other memory technology, compact disk read-only memory (CD-ROM), digital versatile disks (DVD) or other optical disk storage, magnetic cassettes, magnetic tape, magnetic disk storage or other magnetic storage devices, or any other medium that can be used to store the desired information and that can be accessed by computing device 4701.

Although not required, various aspects described herein may be embodied as a method, a data processing system, or as a computer-readable medium storing computer-executable instructions. For example, a computer-readable medium storing instructions to cause a processor to perform steps of a method in accordance with aspects of the disclosed embodiments is contemplated. For example, aspects of the method steps disclosed herein may be executed on a processor on computing device 4701. Such a processor may execute computer-executable instructions stored on a computer-readable medium.

Software may be stored within memory 4715 and/or storage to provide instructions to processor 4703 for enabling computing device 4701 to perform various functions. For example, memory 4715 may store software used by computing device 4701, such as operating system 4717, application programs 4719, and associated database 4721. Also, some or all of the computer executable instructions for computing device 4701 may be embodied in hardware or firmware. Although not shown, RAM 4705 may include one or more applications representing the application data stored in RAM 4705 while computing device 4701 is on and corresponding software applications (e.g., software tasks) are running on computing device 4701.

Communications module 4709 may include a microphone, keypad, touch screen, and/or stylus through which a user of computing device 4701 may provide input, and may also include one or more of a speaker for providing audio output and a video display device for providing textual, audiovisual and/or graphical output. Computing system environment 4700 may also include optical scanners (not shown). Illustrative usages include scanning and converting paper documents, e.g., correspondence, receipts, and the like, to digital files.

Computing device 4701 may operate in a networked environment supporting connections to one or more remote computing devices, such as computing devices 4741, 4751, and 4761. Computing devices 4741, 4751, and 4761 may be personal computing devices or servers that include any or all of the elements described above relative to computing device 4701. Computing device 4761 may be a mobile device (e.g., smart phone) communicating over wireless carrier channel 4771.

The network connections depicted in FIG. 1 may include local area network (LAN) 4725 and wide area network (WAN) 4729, as well as other networks. When used in a LAN networking environment, computing device 101 may be connected to LAN 4725 through a network interface or adapter in communications module 4709. When used in a WAN networking environment, computing device 4701 may include a modem in communications module 4709 or other means for establishing communications over WAN 4729, such as Internet 4731 or other type of computer network. The network connections shown are illustrative and other means of establishing a communications link between the computing devices may be used. Various well-known protocols such as transmission control protocol / Internet protocol (TCP/IP), Ethernet, file transfer protocol (FTP), hypertext transfer protocol (HTTP) and the like may be used, and the system can be operated in a client-server configuration to permit a user to retrieve web pages from a web-based server. Any of various conventional web browsers can be used to display and manipulate data on web pages.

The disclosure is operational with numerous other general purpose or special purpose computing system environments or configurations. Examples of well-known computing systems, environments, and/or configurations that may be suitable for use with the disclosed embodiments include, but are not limited to, personal computers (PCs), server computers, hand-held or laptop devices, smart phones, multiprocessor systems, microprocessor-based systems, set top boxes, programmable consumer electronics, network PCs, minicomputers, mainframe computers, distributed computing environments that include any of the above systems or devices, and the like.

While aspects of the present disclosure have been described in terms of preferred examples, and it will be understood that the disclosure is not limited thereto since modifications may be made to those skilled in the art, particularly in light of the foregoing teachings.

## Claims

1. An apparatus (10) comprising:

   a body comprising a proximal portion (12), a distal portion (14), and a main portion (16) formed between the proximal portion and distal portion;
   at least one ultrasound transducer arranged at the distal portion of the body and configured to scan a region extending away from the main portion of the body; and
   a signal processing unit (32) comprising at least one processor and memory storing instructions;

   **characterised in that**:
   the apparatus (10) is configured for distinguishing nerve tissue from surrounding tissue, and **in that** said instructions, when executed by said at least one processor, cause the at least one processor to:

   generate, based on a signal received from the at least one ultrasound transducer (26), a b-mode scan line;
   determine, based on statistical information of received RF data and an envelope associated with the b-mode scan line, features for use in classifying the b-mode scan line;
   using the determined features and a multivariate classification algorithm, classify the b-mode scan line; and
   based on the classification, determine a presence or an absence of the nerve tissue.

2. The apparatus (10) of claim 1, further comprising:
   an outputting unit configured to communicate with the signal processing unit (32), wherein the outputting unit is further configured to output an indication associated with presence or the absence of the nerve tissue.

3. The apparatus (10) of claim 2, wherein the indication is an audio indication and wherein the indication indicates that the nerve tissue is within a predetermined distance from the distal portion (14).

4. The apparatus (10) of claim 2, wherein the indication comprises a visual identifier of the nerve tissue.

5. The apparatus (10) of claim 1, wherein the at least one ultrasound transducer (26) comprises at least two ultrasound transducers.

6. The apparatus (10) of claim 1, wherein the at least one ultrasound transducer (26) comprises a micro machined array.

7. The apparatus (10) of claim 1, further comprising a glove, wherein a fingertip of the glove comprises the distal portion (14).

8. The apparatus (10) of claim 1, wherein the main portion (16) comprises a conduit arranged along a longitudinal axis (L) of the main portion and configured to receive an instrument.

9. The apparatus (10) of claim 1, wherein the features comprise one or more of: a skewness of the received RF, a mean of the envelope, a variance of the envelope, a skewness of the envelope, a kurtosis of the envelope, a mean of a log of the envelope, a variance of the log of the envelope, a skewness of the log of the envelope, and a kurtosis of the log of the envelope.

10. The apparatus (10) of claim 1, wherein the multivariate classification algorithm comprises a support vector machine (SVM) with a Gaussian kernel.

11. A method for distinguishing nerve tissue from surrounding tissue, comprising:

receiving, at a computing device, a signal from at least one ultrasound transducer (26) arranged at a distal portion (14) of a body, wherein the at least one ultrasound transducer is configured to scan a region extending away from a main portion (16) of the body;
processing, by the computing device, the signal to identify a presence or an absence of the nerve tissue, wherein the processing comprises:

based on the signal, generating a b-mode scan line;
determining, based on statistical information of received RF data and an envelope associated with the b-mode scan line, features for use in classifying the b-mode scan line;

using the determined features and a multivariate classification algorithm, classifying the b-mode scan line; and
based on the classification, determining the presence or the absence of the nerve tissue; and
outputting, by the computing device, an indication associated with the presence or the absence of the nerve tissue.

12. The method of claim 11, wherein the indication comprises a visual identifier of the nerve tissue.

13. The method of claim 11, wherein the at least one ultrasound transducer (26) comprises at least two ultrasound transducers, the method further comprising:
prior to receiving the signal, causing transmitting of a first sound wave by a first ultrasound transducer of the at least two ultrasound transducers and a second sound wave by a second ultrasound transducer of the at least two ultrasound transducers.

14. The method of claim 13, wherein the first sound wave comprises a first frequency and wherein the second sound wave comprises a second frequency different from the first frequency.

15. The method of claim 11, wherein the features comprise one or more of: a skewness of the received RF, a mean of the envelope, a variance of the envelope, a skewness of the envelope, a kurtosis of the envelope, a mean of a log of the envelope, a variance of the log of the envelope, a skewness of the log of the envelope, and a kurtosis of the log of the envelope.

16. The method of claim 11, wherein the multivariate classification algorithm comprises a support vector machine (SVM) with a Gaussian kernel.

17. A non-transitory computer readable medium storing instructions that, when executed by one or more processors, cause the one or more processors to:

receive a signal from at least one ultrasound transducer (26) arranged at a distal portion (14) of a body and configured to scan a region extending away from a main portion (16) of the body;
based on the signal, generate a b-mode scan line;
determine, based on statistical information of received RF data and an envelope associated with the b-mode scan line, features for use in classifying the b-mode scan line;
using the determined features and a multivariate classification algorithm, classify the b-mode scan line;
based on the classification, determine a presence or an absence of a nerve tissue; and

output an indication associated with the presence or the absence of the nerve tissue.

18. The computer readable medium of claim 17, wherein the indication is an audio indication indicating the nerve tissue is within a predetermined distance from the distal portion (14).

19. The computer readable medium of claim 17, wherein the indication comprises a visual identifier of the nerve tissue.

20. The computer readable medium of claim 17, wherein the features comprise one or more of: a skewness of the received RF, a mean of the envelope, a variance of the envelope, a skewness of the envelope, a kurtosis of the envelope, a mean of a log of the envelope, a variance of the log of the envelope, a skewness of the log of the envelope, and a kurtosis of the log of the envelope.

**Patentansprüche**

1. Vorrichtung (10), umfassend:

   einen Körper mit einem proximalen Abschnitt (12), einem distalen Abschnitt (14) und einem Hauptabschnitt (16), der zwischen dem proximalen Abschnitt und dem distalen Abschnitt gebildet ist;
   wenigstens einen Ultraschallwandler, der am distalen Abschnitt des Körpers angeordnet und so ausgelegt ist, dass er einen Bereich abtastet, der sich vom Hauptabschnitt des Körpers weg erstreckt; und
   eine Signalverarbeitungseinheit (32) mit wenigstens einem Prozessor und
   einem Speicher, der Anweisungen speichert; **dadurch gekennzeichnet, dass**:
   die Vorrichtung (10) zum Unterscheiden von Nervengewebe von umgebendem Gewebe ausgelegt ist, und dass die Anweisungen, wenn sie von dem wenigstens einen Prozessor ausgeführt werden, bewirken, dass der wenigstens eine Prozessor:

   basierend auf einem von dem wenigstens einen Ultraschallwandler (26) empfangenen Signale eine b-Modus-Abtastlinie erzeugt;
   basierend auf statistischen Informationen empfangener RF-Daten und einer mit der b-Modus-Abtastlinie verbundenen Hüllkurve Merkmale zur Verwendung bei der Klassifizierung der b-Modus-Abtastlinie bestimmt;
   unter Verwendung der bestimmten Merkmale und eines multivariaten Klassifikationsalgorithmus die b-Modus-Abtastlinie zu klassifizieren; und
   basierend auf der Klassifizierung ein Vorhandensein oder ein Nichtvorhandensein des Nervengewebes bestimmt.

2. Vorrichtung (10) nach Anspruch 1, ferner umfassend:
   eine Ausgabeeinheit, die so ausgelegt ist, dass sie mit der Signalverarbeitungseinheit (32) kommuniziert, wobei die Ausgabeeinheit ferner so ausgelegt ist, dass sie eine mit dem Vorhandensein oder Nichtvorhandensein des Nervengewebes verbundene Anzeige ausgibt.

3. Vorrichtung (10) nach Anspruch 2, wobei die Anzeige eine Audioanzeige ist und wobei die Anzeige angibt, dass sich das Nervengewebe innerhalb eines vorbestimmten Abstands vom distalen Abschnitt (14) befindet.

4. Vorrichtung (10) nach Anspruch 2, wobei die Anzeige eine visuelle Kennung des Nervengewebes umfasst.

5. Vorrichtung (10) nach Anspruch 1, wobei der wenigstens eine Ultraschallwandler (26) wenigstens zwei Ultraschallwandler umfasst.

6. Vorrichtung (10) nach Anspruch 1, wobei der wenigstens eine Ultraschallwandler (26) ein mikrobearbeitetes Array umfasst.

7. Vorrichtung (10) nach Anspruch 1, die ferner einen Handschuh umfasst, wobei eine Fingerspitze des Handschuhs den distalen Abschnitt (14) umfasst.

8. Vorrichtung (10) nach Anspruch 1, wobei der Hauptabschnitt (16) eine Leitung umfasst, die entlang einer Längsachse (L) des Hauptabschnitts angeordnet und zur Aufnahme eines Instruments ausgelegt ist.

9. Vorrichtung (10) nach Anspruch 1, wobei die Merkmale eines oder mehrere der folgenden Merkmale umfassen: eine Schiefe (skewness) der empfangenen RF, einen Mittelwert der Hüllkurve, eine Varianz der Hüllkurve, eine Schiefe (skewness) der Hüllkurve, eine Kurtosis der Hüllkurve, einen Mittelwert eines Logarithmus der Hüllkurve, eine Varianz des Logarithmus der Hüllkurve, eine Schiefe (skewness) des Logarithmus der Hüllkurve und eine Kurtosis des Logarithmus der Hüllkurve.

10. Vorrichtung (10) nach Anspruch 1, wobei der multivariate Klassifikationsalgorithmus eine Support Vector Machine (SVM) mit einem Gauß-Kernel umfasst.

11. Verfahren zur Unterscheidung von Nervengewebe von umgebendem Gewebe, das umfasst:

> Empfangen eines Signals von wenigstens einem Ultraschallwandler (26), der an einem distalen Abschnitt (14) eines Körpers angeordnet ist, an einer Rechenvorrichtung, wobei der wenigstens eine Ultraschallwandler so ausgelegt ist, dass er einen Bereich abtastet, der sich von einem Hauptabschnitt (16) des Körpers weg erstreckt;
> Verarbeiten des Signals durch die Rechenvorrichtung, um ein Vorhandensein oder ein Nichtvorhandensein des Nervengewebes zu identifizieren, wobei die Verarbeitung umfasst:
>
>> Erzeugen basierend auf dem Signal einer b-Modus-Abtastlinie;
>> Bestimmen von Merkmalen zur Verwendung bei der Klassifizierung der b-Modus-Abtastlinie, basierend auf statistischen Informationen empfangener RF-Daten und einer mit der b-Modus-Abtastlinie verbundenen Hüllkurve;
>> Klassifizierung der b-Modus-Abtastlinie unter Verwendung der bestimmten Merkmale und eines multivariaten Klassifikationsalgorithmus; und
>> Bestimmen des Vorhandenseins oder des Nichtvorhandenseins des Nervengewebes basierend auf der Klassifizierung; und
>> Ausgabe einer mit dem Vorhandensein oder Nichtvorhandensein des Nervengewebes verbundenen Anzeige durch die Recheneinrichtung.

12. Verfahren nach Anspruch 11, wobei die Anzeige eine visuelle Kennung des Nervengewebes umfasst.

13. Verfahren nach Anspruch 11, wobei der wenigstens eine Ultraschallwandler (26) wenigstens zwei Ultraschallwandler umfasst, wobei das Verfahren ferner umfasst:
vor dem Empfangen des Signals, Bewirken des Sendens einer ersten Schallwelle durch einen ersten Ultraschallwandler der wenigstens zwei Ultraschallwandler und einer zweiten Schallwelle durch einen zweiten Ultraschallwandler der wenigstens zwei Ultraschallwandler.

14. Verfahren nach Anspruch 13, wobei die erste Schallwelle eine erste Frequenz umfasst und wobei die zweite Schallwelle eine zweite Frequenz umfasst, die sich von der ersten Frequenz unterscheidet.

15. Verfahren nach Anspruch 11, wobei die Merkmale eines oder mehreres vom Folgenden umfassen: eine Schiefe (skewness) der empfangenen HF, einen Mittelwert der Hüllkurve, eine Varianz der Hüllkurve, eine Schiefe (skewness) der Hüllkurve, eine Kurtosis der Hüllkurve, einen Mittelwert eines Logarithmus der Hüllkurve, eine Varianz des Logarithmus der Hüllkurve, eine Schiefe (skewness) des Logarithmus der Hüllkurve, und eine Kurtosis des Logarithmus der Hüllkurve.

16. Verfahren nach Anspruch 11, wobei der multivariate Klassifikationsalgorithmus eine Support Vector Machine (SVM) mit einem Gauß-Kernel umfasst.

17. Nicht-transitorisches computerlesbares Medium, das Befehle speichert, die, wenn sie von einem oder mehreren Prozessoren ausgeführt werden, bewirken, dass der eine oder die mehreren Prozessoren:

> ein Signal von wenigstens einem Ultraschallwandler (26) empfängt, der an einem distalen Abschnitt (14) eines Körpers angeordnet und so ausgelegt ist, dass er einen Bereich abtastet, der sich von einem Hauptabschnitt (16) des Körpers weg erstreckt;
> basierend auf dem Signal eine b-Modus-Abtastlinie erzeugt;
> basierend auf statistischen Informationen empfangener HF-Daten und einer mit der b-Modus-Abtastlinie verbundenen Hüllkurve Merkmale zur Verwendung bei der Klassifizierung der b-Modus-Abtastlinie bestimmt;
> unter Verwendung der bestimmten Merkmale und einem multivariaten Klassifikationsalgorithmus die b-Modus-Abtastlinie klassifiziert;
> auf der Grundlage der Klassifizierung das Vorhandensein oder die Abwesenheit eines Nervengewebes bestimmt; und eine Anzeige ausgibt, die mit dem Vorhandensein oder dem Nichtvorhandensein des Nervengewebes verbunden ist.

18. Computerlesbares Medium nach Anspruch 17, wobei die Anzeige eine Audioanzeige ist, die angibt, dass sich das Nervengewebe innerhalb eines vorbestimmten Abstands vom distalen Abschnitt (14) befindet.

19. Computerlesbares Medium nach Anspruch 17, wobei die Anzeige eine visuelle Kennung des Nervengewebes umfasst.

20. Computerlesbares Medium nach Anspruch 17, wobei die Merkmale eines oder mehreres von Folgendem umfassen: eine Schiefe (skewness) der empfangenen HF, einen Mittelwert der Hüllkurve, eine Varianz der Hüllkurve, eine Schiefe (skewness) der Hüllkurve, eine Kurtosis der Hüllkurve, einen Mittelwert eines Logarithmus der Hüllkurve, eine Varianz des Logarithmus der Hüllkurve, eine Schiefe (skewness) des Logarithmus der Hüllkurve und eine Kurtosis des Logarithmus der Hüllkurve.

## Revendications

1. Appareil (10) comprenant :

   un corps qui comprend une partie proximale (12), une partie distale (14) et une partie principale (16) qui est formée entre la partie proximale et la partie distale ;
   au moins un transducteur à ultrasons qui est agencé au niveau de la partie distale du corps et qui est configuré pour balayer une région qui est étendue à distance par rapport à la partie principale du corps ; et
   une unité de traitement de signal (32) qui comprend au moins un processeur et une mémoire qui stocke des instructions ;
   **caractérisé en ce que** :
   l'appareil (10) est configuré pour distinguer un tissu nerveux d'un tissu avoisinant, et **en ce que** lesdites instructions, lorsqu'elles sont exécutées par ledit au moins processeur, forcent l'au moins un processeur à :

   générer, sur la base d'un signal qui est reçu depuis l'au moins un transducteur à ultrasons (26), une ligne de balayage de mode b ;
   déterminer, sur la base d'une information statistique de données RF reçues et d'une enveloppe qui est associée à la ligne de balayage de mode b, des caractéristiques dans le but de leur utilisation au niveau de la classification de la ligne de balayage de mode b ;
   en utilisant les caractéristiques déterminées et un algorithme de classification à multiples variables, classifier la ligne de balayage de mode b ; et
   sur la base de la classification, déterminer une présence ou une absence du tissu nerveux.

2. Appareil (10) selon la revendication 1, comprenant en outre :
   une unité d'émission en sortie qui est configurée pour communiquer avec l'unité de traitement de signal (32), dans lequel l'unité d'émission en sortie est en outre configurée pour émettre en sortie une indication qui est associée à la présence ou à l'absence du tissu nerveux.

3. Appareil (10) selon la revendication 2, dans lequel l'indication est une indication audio et dans lequel l'indication indique que le tissu nerveux est à l'intérieur d'une distance prédéterminée par rapport à la partie distale (14).

4. Appareil (10) selon la revendication 2, dans lequel l'indication comprend un identifiant visuel du tissu nerveux.

5. Appareil (10) selon la revendication 1, dans lequel l'au moins un transducteur à ultrasons (26) comprend au moins deux transducteurs à ultrasons.

6. Appareil (10) selon la revendication 1, dans lequel l'au moins un transducteur à ultrasons (26) comprend un réseau micro-usiné.

7. Appareil (10) selon la revendication 1, comprenant en outre un gant, dans lequel un doigtier du gant comprend la partie distale (14).

8. Appareil (10) selon la revendication 1, dans lequel la partie principale (16) comprend un conduit qui est agencé suivant un axe longitudinal (L) de la partie principale et qui est configuré pour recevoir un instrument.

9. Appareil (10) selon la revendication 1, dans lequel les caractéristiques comprennent un ou plusieurs paramètre(s) pris parmi : une dissymétrie de la fréquence radio/RF reçue, une moyenne de l'enveloppe, une variance de l'enveloppe, une dissymétrie de l'enveloppe, un coefficient d'aplatissement de l'enveloppe, une moyenne d'un logarithme de l'enveloppe, une variance du logarithme de l'enveloppe, une dissymétrie du logarithme de l'enveloppe et un coefficient d'aplatissement du logarithme de l'enveloppe.

10. Appareil (10) selon la revendication 1, dans lequel l'algorithme de classification à multiples variables comprend une machine à vecteurs de support (SVM) avec un noyau gaussien.

11. Procédé pour distinguer un tissu nerveux d'un tissu avoisinant, comprenant :

    la réception, au niveau d'un dispositif informatique, d'un signal en provenance d'au moins un transducteur à ultrasons (26) qui est agencé au niveau d'une partie distale (14) d'un corps, dans lequel l'au moins un transducteur à ultrasons est configuré pour balayer une région qui est étendue à distance par rapport à une partie principale (16) du corps ;
    le traitement, par le dispositif informatique, du signal afin d'identifier une présence ou une absence du tissu nerveux, dans lequel le traitement comprend :

    sur la base du signal, la génération d'une ligne de balayage de mode b ;

la détermination, sur la base d'une information statistique de données RF reçues et d'une enveloppe qui est associée à la ligne de balayage de mode b, de caractéristiques dans le but de leur utilisation au niveau de la classification de la ligne de balayage de mode b ;

en utilisant les caractéristiques déterminées et un algorithme de classification à multiples variables, la classification de la ligne de balayage de mode b ; et

sur la base de la classification, la détermination de la présence ou de l'absence du tissu nerveux ; et

l'émission en sortie, par le dispositif informatique, d'une indication qui est associée à la présence ou à l'absence du tissu nerveux.

12. Procédé selon la revendication 11, dans lequel l'indication comprend un identifiant visuel du tissu nerveux.

13. Procédé selon la revendication 11, dans lequel l'au moins un transducteur à ultrasons (26) comprend au moins deux transducteurs à ultrasons, le procédé comprenant en outre :

avant la réception du signal, le fait de provoquer l'émission d'une première onde sonore par un premier transducteur à ultrasons des au moins deux transducteurs à ultrasons et d'une seconde onde sonore par un second transducteur à ultrasons des au moins deux transducteurs à ultrasons.

14. Procédé selon la revendication 13, dans lequel la première onde sonore comprend une première fréquence et dans lequel la seconde onde sonore comprend une seconde fréquence qui est différente de la première fréquence.

15. Procédé selon la revendication 11, dans lequel les caractéristiques comprennent un ou plusieurs paramètre(s) pris parmi : une dissymétrie de la fréquence radio/RF reçue, une moyenne de l'enveloppe, une variance de l'enveloppe, une dissymétrie de l'enveloppe, un coefficient d'aplatissement de l'enveloppe, une moyenne d'un logarithme de l'enveloppe, une variance du logarithme de l'enveloppe, une dissymétrie du logarithme de l'enveloppe et un coefficient d'aplatissement du logarithme de l'enveloppe.

16. Procédé selon la revendication 11, dans lequel l'algorithme de classification à multiples variables comprend une machine à vecteurs de support (SVM) avec un noyau gaussien.

17. Support non transitoire pouvant être lu par un ordinateur et stockant des instructions qui, lorsqu'elles sont exécutées par un ou plusieurs processeur(s), forcent les un ou plusieurs processeurs à :

recevoir un signal en provenance d'au moins un transducteur à ultrasons (26) qui est agencé au niveau d'une partie distale (14) d'un corps et qui est configuré pour balayer une région qui est étendue à distance par rapport à une partie principale (16) du corps ;

sur la base du signal, générer une ligne de balayage de mode b ;

déterminer, sur la base d'une information statistique de données RF reçues et d'une enveloppe qui est associée à la ligne de balayage de mode b, des caractéristiques dans le but de leur utilisation au niveau de la classification de la ligne de balayage de mode b ;

en utilisant les caractéristiques déterminées et un algorithme de classification à multiples variables, classifier la ligne de balayage de mode b ;

sur la base de la classification, déterminer une présence ou une absence d'un tissu nerveux ; et émettre en sortie une indication qui est associée à la présence ou à l'absence du tissu nerveux.

18. Support pouvant être lu par un ordinateur selon la revendication 17, dans lequel l'indication est une indication audio qui indique que le tissu nerveux est à l'intérieur d'une distance prédéterminée par rapport à la partie distale (14).

19. Support pouvant être lu par un ordinateur selon la revendication 17, dans lequel l'indication comprend un identifiant visuel du tissu nerveux.

20. Support pouvant être lu par un ordinateur selon la revendication 17, dans lequel les caractéristiques comprennent un ou plusieurs paramètre(s) pris parmi : une dissymétrie de la fréquence radio/RF reçue, une moyenne de l'enveloppe, une variance de l'enveloppe, une dissymétrie de l'enveloppe, un coefficient d'aplatissement de l'enveloppe, une moyenne d'un logarithme de l'enveloppe, une variance du logarithme de l'enveloppe, une dissymétrie du logarithme de l'enveloppe et un coefficient d'aplatissement du logarithme de l'enveloppe.

**FIG. 1**

**FIG. 2**

*FIG. 3*

Single Element Focused

26

Micro-machined array (CMUT)

26

~3 mm

Requires multiple channels or
integrated circuit in transducer assembly

*FIG. 4*

EP 3 193 726 B1

## FIG. 5

## FIG. 6

FIG. 7

26 Scanning Region

D

Transducer Width = Scanning Width

FIG.8

10 44

20 26 44

FIG.9

20 40

α

L

42

FIG.11

20 40

α₁

α₂

L

42

FIG.12

FIG. 10

1300

1350

*FIG.13*

Lateral distance in mm

Time (US)

*FIG.14*

1400

1410

FIG.15

1500

1600

FIG.16

*FIG.17*

1710 1720 1700

1800

*FIG.18*

FIG. 19

FIG. 20

FIG. 21

FIG. 22

FIG. 23

FIG. 24

FIG. 25

*FIG. 26*

FIG. 27

FIG. 28

*FIG. 29*

*FIG. 30*

*FIG. 31*

**FIG. 32**

**FIG. 33**

*FIG. 34*

*FIG. 36*

*FIG. 37*

*FIG. 35*

**FIG. 38**

**FIG. 39**

**FIG. 40**

Unclipped data

Scan Line Energy

4100

FIG. 41

FIG. 42

EP 3 193 726 B1

Postfiltered Image

Prefiltered Image

Low Frequency Ringing

4300

*FIG. 43*

FIG. 44

EP 3 193 726 B1

FIG. 45:

| FIG. 45A | FIG. 45B |

EP 3 193 726 B1

4500

FIG. 45A

FIG. 45B

FIG. 46

4600

EP 3 193 726 B1

FIG. 47

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012197124 A1
- US 7920922 B

- US 6652452 B